(19) Europäisches Patentamt
European Patent Office
Office européen des brevets

(11) **EP 4 094 776 A1**

(12) **EUROPEAN PATENT APPLICATION**
published in accordance with Art. 153(4) EPC

(43) Date of publication:
**30.11.2022 Bulletin 2022/48**

(21) Application number: 21753144.1

(22) Date of filing: **08.02.2021**

(51) International Patent Classification (IPC):
*A61K 38/48* (2006.01)     *A61K 38/49* (2006.01)
*A61K 38/36* (2006.01)     *A61P 31/00* (2006.01)
*A61P 31/14* (2006.01)     *A61P 11/00* (2006.01)

(52) Cooperative Patent Classification (CPC):
**A61K 38/36; A61K 38/48; A61K 38/49;
A61P 11/00; A61P 31/00; A61P 31/14**

(86) International application number:
**PCT/CN2021/076035**

(87) International publication number:
**WO 2021/160092 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.02.2020   CN 202010086980**

(71) Applicant: **Talengen International Limited
Hong Kong 999077 (HK)**

(72) Inventor: **LI, Jinan
Shenzhen, Guangdong 518020 (CN)**

(74) Representative: **karo IP
karo IP Patentanwälte
Kahlhöfer Rößler Kreuels PartG mbB
Postfach 32 01 02
40416 Düsseldorf (DE)**

Remarks:
The complete document including Reference
Table(s) and the Sequence Listing(s) can be
downloaded from the EPO website

(54) **METHOD AND DRUG FOR TREATING VIRAL PNEUMONIA**

(57)     Provided is a method for treating viral pneumonia, comprising: administrating a therapeutically effective amount of a component of plasminogen activation pathway to a subject. Also provided are a medicament, a phar- maceutical composition, a product, and a kit which comprise a component of plasminogen activation pathway for treating viral pneumonia.

EP 4 094 776 A1

**Description**

TECHNICAL FIELD

The present application relates to a method for preventing or treating viral pneumonia, such as coronavirus-infected pneumonia and complications thereof, comprising: administering an effective amount of a component of the plasminogen activation pathway or a related compound thereof (e.g., plasminogen) to a subject to alleviate lung inflammation, increase blood oxygen saturation, treat pneumonia and prevent related complications such as pulmonary fibrosis.

BACKGROUND

Coronavirus (CoV) is a positive single-stranded RNA virus with envelope, belonging to *Coronaviridae* family, and can be divided into four genera according to their genotypes: alpha coronavirus ($\alpha$-CoV), beta coronavirus ($\beta$-CoV), gamma coronavirus ($\gamma$-CoV), delta coronavirus ($\delta$-CoV); wherein $\alpha$-CoV and $\beta$-CoV are more susceptible to mammals (CUI J, Li F, SHI Z L. Origin and evolution of pathogenic coronaviruses[J]. Naturereviews. Micorbiology, 2019, 17(3): 181-192).

Severe acute respiratory syndrome coronavirus (SARS-CoV), which outbroke in China in 2003 and caused severe acute respiratory syndrome (SARS) characterized by atypical pneumonia, belongs to $\beta$-CoV. A new human coronavirus (hCoVEMC/2012) similar to SARS-CoV appeared in the Middle East and South Korea in 2012, and caused middle east respiratory syndrome (MERS) in humans. In 2013, the World Health Organization named its pathogen as Middle East respiratory syndrome coronavirus (MERS-CoV). The disease is similar to SARS, but it is more likely to cause other complications, such as liver and kidney failure, thus the mortality rate of patients is higher.

SARS virus has extensive tissue tropism, invades various tissues and organs, and causes damage to various tissues and organs. A patient has the symptoms including pulmonary congestion, hemorrhage, edema and severe diffuse alveolar damage, hyaline membrane formation, alveolar epithelial hyperplasia, inflammatory infiltration of pulmonary interstitial mononuclear cells, shedding of interalveolar lung cells, and pulmonary interstitial fibrosis.

The 2019 novel coronavirus, which was first discovered in December 2019 in Wuhan, Hubei Province and spread rapidly in a short period of time. It was officially named 2019-nCoV by WHO on January 12, 2020 (also referred to as COVID-19 in this application), belongs to $\beta$-genus coronavirus and its genetic characteristics are significantly different from SARS-CoV and MERS-CoV, and closer to the bat SARS-like coronavirus bat-SL-CoV ZC45 and bat-SL-CoV ZXC21 (CHEN Y, LIU Q, GUO D. Emerging coronaviruses: genome structure, replication, and pathogenesis [J]. Journal of medical virology, 2020).

2019-nCoV has high infectivity, and the population is generally susceptible to it, the main symptoms manifest as lower respiratory tract infection, such as dry cough, fever, dyspnea, etc. In severe cases, it can even cause acute respiratory distress syndrome (ARDS) and sepsis (Clinical management of severe acute respiratory infection when novel coronavirus (2019-nCoV) infection is suspected: interim guidance.28January 2020.WHO/nCoV/Clinical/2020.2).

Novel coronavirus pneumonia can be divided into three stages based on pathological staging: early stage, advanced stage and severe stage. At early stage, the disease is limited, and the symptoms manifest as single or multiple ground-glass opacity (GGO) nodules, patches or sheet shadows; in the advanced stage, the disease progresses, the lesions increase and expand in scope, GGO coexists with consolidation or strip shadows, partial consolidation or columnar thickening of the bronchi in structural distortion shadows; in severe stage, diffuse lesions in both lungs, extensive exudation and consolidation of lung parenchyma, mainly consolidation shadows, distorted lung structure, bronchiectasis, sub-segmental atelectasis, with "white lung" in severe cases are observed (SHI Heshui, HAN Xiaoyu, FAN Yanqing, LIANG Bo, YANG Fan, HAN Ping, ZHENG Chuansheng. Clinical features and imaging manifestations of pneumonia caused by novel coronavirus (2019-nCoV) infection. Journal of Clinical Radiology. https://doi.org/10.13437/j.cnki.jcr.20200206.002).

SUMMARY OF THE APPLICATION

The present application finds that plasminogen can significantly alleviate pneumonia (including viral pneumonia, such as 2019-nCoV viral pneumonia) and improve lung ventilation function in patients with pulmonary fibrosis, increase blood oxygen saturation and improve symptoms of dyspnea, thereby treating pneumonia and pulmonary fibrosis -related diseases.

Particularly, the present application relates to the following items:

1. In one aspect, the application relates to a method for preventing and treating pneumonia, comprising: administrating to a subject a therapeutically effective amount of one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, the present application relates to use of one or more compounds in the preparation of a medicament for preventing and treating pneumonia, wherein said one or more compounds are selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, the present application relates to use of one or more compounds in the prevention and treatment of pneumonia, wherein said one or more compounds are selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, the present application relates to a medicament for the prevention and treatment of pneumonia, which comprises one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

2. The method, use or medicament according to item 1, wherein the component of plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains and protease domains of plasminogen and plasmin, mini-plasminogen, mini-plasmin, micro-plasminogen, micro-plasmin, delta-plasminogen, delta-plasmin, an activator of plasminogen, tPA and uPA.

3. The method, use or medicament according to item 1, wherein the antagonist of the fibrinolysis inhibitor is an inhibitor of PAI-1, complement C1 inhibitor, $\alpha2$ antiplasmin or $\alpha2$ macroglobulin, e.g., an antibody.

4. The method, use or medicament according to any one of items 1-3, wherein the pneumonia is bacterial pneumonia, viral pneumonia, mycoplasmal pneumonia, chlamydia pneumonia, fungal pneumonia, or rickettsial pneumonia.

5. The method, use or medicament according to any one of items 1-3, wherein the pneumonia is caused by a non-infectious factor. In some embodiments, the pneumonia is caused by radiation. In some embodiments, the pneumonia is caused by inhalation of toxic gases, e.g., a pneumonia caused by dust deposition such as haze. In some embodiments, the pneumonia is caused by an autoimmune disease (e.g., systemic sclerosis). In some embodiments, the pneumonia is caused by allergies, such as asthma. In some embodiments, the pneumonia is caused by a toxic compound (e.g., monocrotaline or paraquat).

6. The method, use or medicament according to item 4, wherein the pneumonia is coronavirus pneumonia.

7. The method, use or medicament according to item 6, wherein the pneumonia is 2019-nCoV infectious pneumonia.

In some embodiments, the pneumonia is 2019-nCoV infectious pneumonia, and the plasminogen has one or more effects selected from the group consisting of: reducing lung tissue damage, reducing lung inflammation, reducing lung fibrin deposition, improving lung function, increasing blood oxygen saturation, reducing blood pressure, improving cardiac function, and improving general physical condition in the subject with 2019-nCoV pneumonia.

8. The method, use or medicament according to any one of items 1-7, wherein the compound has one or more effects selected from the group consisting of: reducing lung tissue inflammation, reducing lung tissue inflammatory exudation, reducing lung tissue fibrin deposition, reducing lung tissue fibrosis, reducing lung tissue apoptosis, improving ventilation function, and increasing blood oxygen saturation.

9. The method, use or medicament according to any one of items 1 to 8, wherein the plasminogen has at least 75%, 80%, 85%, 90%, 95%, 96%, 97%, 98% or 99% sequence identity with SEQ ID NO: 2 and still has plasminogen activity, such as proteolytic activity, lysine binding activity, or both proteolytic activity and lysine binding activity.

10. The method, use or medicament according to any one of items 1 to 8, wherein the plasminogen comprises an amino acid sequence having at least 75%, 80%, 85%, 90%, 95%, 96%, 97%, 98% or 99% sequence identity with the active fragment of plasminogen represented by SEQ ID NO: 14, and has the proteolytic activity or lysine binding activity of plasminogen. In some embodiments, the plasminogen comprises the active fragment of plasminogen represented by SEQ ID NO: 14 and has the proteolytic activity of plasminogen.

11. The method, use or medicament according to any one of items 1-8, wherein the plasminogen is a conservative substitution variant of the plasminogen of SEQ ID NO: 2. In some embodiments, the plasminogen is selected from the group consisting of: Glu-plasminogen, Lys-plasminogen, mini-plasminogen, micro-plasminogen, delta-plasminogen, or a variant thereof retaining plasminogen activity. In some embodiments, the plasminogen comprises or consists of any amino acid sequence selected from SEQ ID NO: 2, 4, 6, 8, 10, 12, or 14.

12. The method, use or medicament according to any one of items 1-8, wherein the plasminogen is natural or synthetic human plasminogen, or a variant or fragment thereof still retaining plasminogen activity.

13. The method, use or medicament according to any one of items 1-12, wherein the compound is used in combination with one or more other therapeutic methods or medicaments. In some embodiments, the other therapeutic method is an extracorporeal assisted breathing method, such as a breathing machine assisted breathing method.

14. The method, use or medicament according to item 13, wherein the other medicament is one or more medicaments selected from the group consisting of: antiviral medicament, antibiotic, immunomodulator, hormonal medicament (e.g., steroid hormone), vaccine, and disease-associated neutralizing antibody.

15. The method, use or medicament according to any one of items 1-14, wherein the compound is administered by one or more routes or means selected from the group consisting of: nasal inhalation, aerosol inhalation, nasal drop, ear drop, eye drop, intravenous administration, intraperitoneal administration, subcutaneous administration, intracranial administration, intrathecal administration, intramuscular administration and intrarectal administration.

In any of the above embodiments of the application, the plasminogen may have at least 75%, 80%, 85%, 90%, 95%, 96%, 97%, 98% or 99% sequence identity with SEQ ID NO: 2, 6, 8, 10 or 12, and still have plasminogen activity, e.g., proteolytic activity, lysine binding activity, or both proteolytic activity and lysine binding activity. In some embodiments, the plasminogen is a protein with addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid based on SEQ ID NO: 2, 6, 8, 10 or 12, and still has plasminogen activity.

In some embodiments, the plasminogen is a protein comprising an active fragment of plasminogen and still having plasminogen activity. In some embodiments, the plasminogen is selected from the group consisting of: Glu-plasminogen, Lys-plasminogen, mini-plasminogen, micro-plasminogen, delta-plasminogen, or variants thereof retaining plasminogen activity. In some embodiments, the plasminogen is natural or synthetic human plasminogen, or a variant or fragment thereof still retaining plasminogen activity. In some embodiments, the plasminogen is a human plasminogen ortholog from a primate or rodent, or a variant or fragment thereof still retaining plasminogen activity. In some embodiments, the amino acid sequence of the plasminogen is represented by SEQ ID NO: 2, 6, 8, 10 or 12. In some embodiments, the plasminogen is human natural plasminogen.

In some embodiments, the subject is a human. In some embodiments, the subject is deficient or lacking in plasminogen. In some embodiments, the lack or deficiency of plasminogen is congenital, secondary and/or local.

In some embodiments, the pharmaceutical composition comprises a pharmaceutically acceptable carrier and plasminogen for use in the above methods. In some embodiments, the kit may be a prophylactic or therapeutic kit, comprising: (i) plasminogen for use in the above methods, and (ii) means for delivering the plasminogen to the subject. In some embodiments, the means is a syringe or a vial. In some embodiments, the kit further comprises a label or instructions for administrating the plasminogen to the subject to perform any of the above methods.

In some embodiments, the product comprises: a container comprising a label; and further comprises (i) plasminogen for use in the above method or a pharmaceutical composition comprising plasminogen, wherein the label instructs the administration of the plasminogen or composition to the subject to perform any of the above methods.

In some embodiments, the kit or product further comprises one or more additional means or containers containing other medicaments.

In some embodiments of the above methods, the plasminogen is administrated by systemic or topical administration for therapy, preferably by one or more routes or means selected from the group consisting of: nasal inhalation, aerosol inhalation, nasal drop, ear drop, eye drop, intravenous administration, intraperitoneal administration, subcutaneous administration, intracranial administration, intrathecal administration, intramuscular administration and intrarectal administration. In some embodiments of the above methods, the plasminogen is administrated in combination with a suitable polypeptide carrier or a stabilizer. In some embodiments of the above methods, the plasminogen is administrated per day at the amount of 0.0001-2000 mg/kg, 0.001-800 mg/kg, 0.01-600 mg/kg, 0.1-400 mg/kg, 1-200 mg/kg, 1-100 mg/kg, or 10-100mg/kg (by per kilogram of body weight); or 0.0001-2000 $mg/cm^2$, 0.001-800 $mg/cm^2$, 0.01-600 $mg/cm^2$, 0.1-400 $mg/cm^2$, 1-200 $mg/cm^2$, 1-100 $mg/cm^2$, or 10-100 $mg/cm^2$ (by per square centimeter of body surface area), preferably repeating at least once, and preferably administrating at least daily.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

BRIEF DESCRIPTION OF THE DRAWINGS

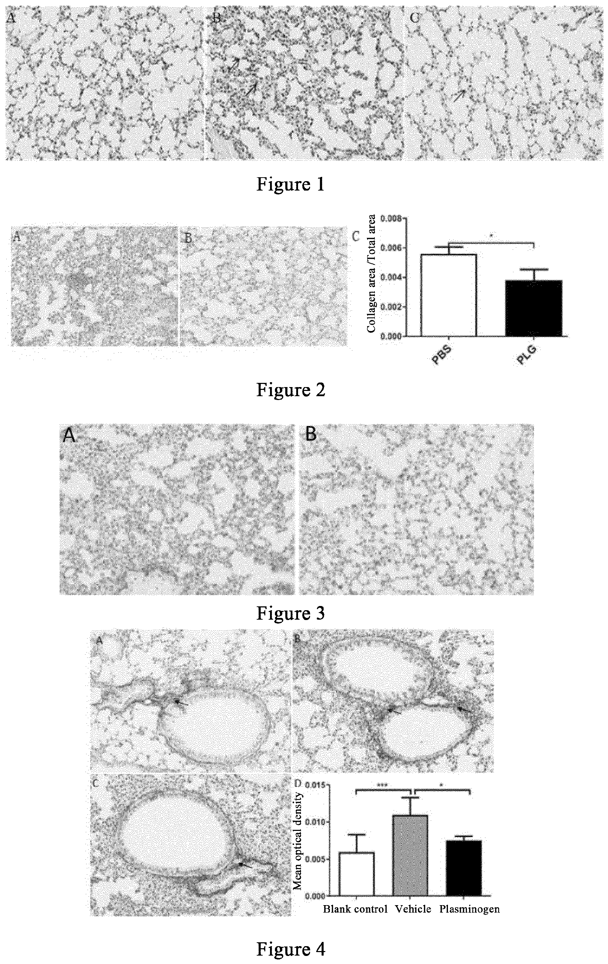

Figs. 1A-C show the representative images of Sirius red staining of lung after administrating plasminogen to the mice of monocrotaline-induced pulmonary hypertension model for 28 days. A is the blank control group, B is the control group in which the mice are given the vehicle PBS (*hereinafter referred to as* vehicle PBS control group, or vehicle group), and C is the group in which the mice are given the plasminogen (*hereinafter referred to as* plasminogen group). The results show that there is basically no collagen deposition in the lungs of the mice in the blank control group, and the collagen deposition (marked by arrows) in the lung tissue of the mice in the plasminogen group is significantly less than that in the vehicle PBS control group. It indicates that plasminogen can significantly reduce the fibrosis of the lungs of the mice of monocrotaline-induced pulmonary hypertension model.

Fig 2A-C show the representative images of Sirius red staining of lung after administrating plasminogen to the mice of bleomycin-induced systemic sclerosis model for 21 days. A is the vehicle PBS control group, B is the plasminogen group, and C is the result of quantitative analysis. The results show that in the mice of the bleomycin-induced systemic sclerosis model, the degree of pulmonary fibrosis in the mice in the PBS group is higher than that in the plasminogen group; the shape of the alveolar wall in the lungs of the mice in the plasminogen group is close to normalization, the level of inflammatory cells is significantly reduced, and the degree of fibrosis is significantly lower than that in the vehicle PBS group, and the statistical difference is significant (* means $P<0.05$).

Figs. 3A-B show the observation results of Sirius red staining of lung after administrating plasminogen to the poisoned mice induced by paraquat for 14 days. A is the vehicle PBS control group, and B is the plasminogen group. The results show that the collagen fibers deposition in the plasminogen group is significantly less than that in the vehicle PBS control group. This indicates that plasminogen can reduce the pulmonary fibrosis caused by paraquat poisoning.

Figs. 4A-D show the results of Sirius red staining of lung after administrating plasminogen to the mice of LPS-induced pneumonia model for 14 days. A is the blank control group, B is the vehicle control group, C is the plasminogen group, and D is the result of quantitative analysis. The results show that the lung tissue of the mice in the blank control group has a certain amount of collagen deposition (marked by arrows), the collagen deposition in the lung tissue of the mice in the vehicle group is significantly increased, and the collagen deposition in the lung tissue of the mice in the plasminogen group is significantly less than that in the vehicle group, and the statistical difference is significant (* means $P<0.05$). It indicates that plasminogen can reduce the collagen deposition in the lungs of the model mice with pneumonia and improve the pulmonary fibrosis caused by pneumonia.

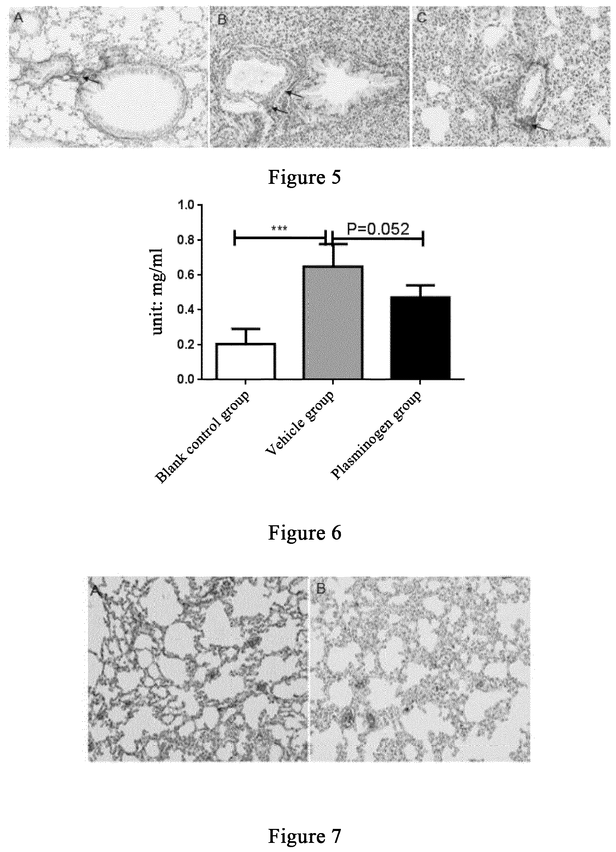

Figs. 5A-C show the results of Sirius red staining of lung in the model mice of LPS-induced pneumonia with administration of plasminogen in advance for 3 days. A is the blank control group, B is the vehicle control group, and C is the plasminogen group. The results show that the lung tissue of the blank control group has a certain amount of collagen deposition (marked by arrows), the collagen deposition in the lung tissue of the mice in the vehicle group is significantly increased, and the collagen deposition in the lung tissue of the mice in the plasminogen group is significantly less than that in the vehicle group. It indicates that the administration of plasminogen in advance can reduce the collagen deposition in lungs of the model mice with pneumonia, and improve the pulmonary fibrosis caused by pneumonia.

Fig. 6 shows the detection result of total protein in lung lavage fluid after administrating plasminogen to the model mice of LPS-induced pneumonia for 7 days. The results show that there is a certain amount of total protein in the lung lavage fluid of the mice in the blank control group, and the total protein level in the lung lavage fluid of the mice in the vehicle group is significantly higher than that of the blank control group (*** means $P<0.001$), while the total protein level in the lung lavage fluid of the plasminogen group is significantly lower than that of the vehicle group, and the statistical difference is close to significance ($P=0.052$). It indicates that plasminogen can reduce the total protein level in lung lavage fluid of the model mice with pneumonia.

Fig. 7 shows the result of immunohistochemical staining of lung fibrin after administrating plasminogen to the model mice of LPS-induced pneumonia for 3 days. A is the vehicle control group, and B is the plasminogen group. The results show that the level of fibrin deposition in the lung tissue of the mice in the vehicle group is significantly higher than that in the plasminogen group. It indicates that plasminogen can reduce the deposition of lung fibrin in the model mice of pneumonia.

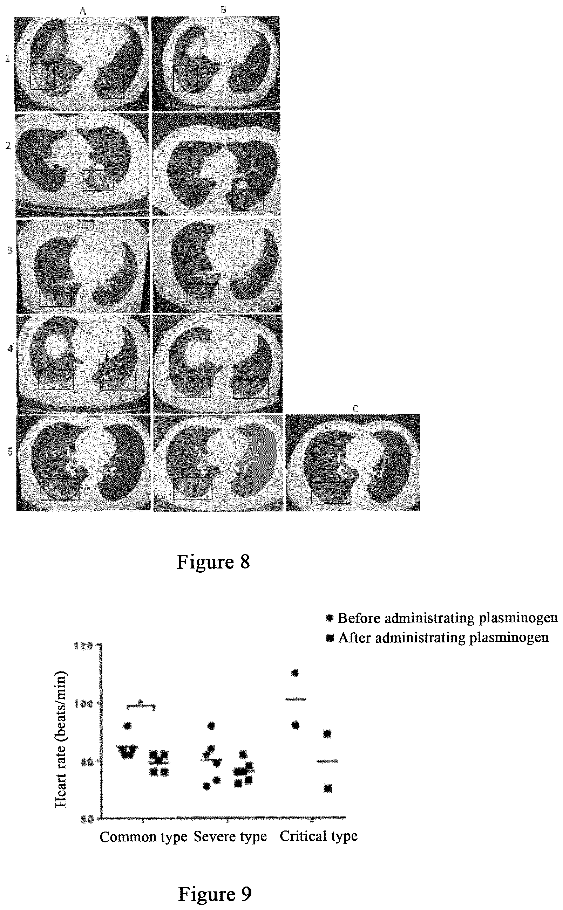

Fig. 8 shows the high-resolution CT images of patients with common COVID-19. The patient's ID is displayed on the left. Column A: CT images of the chest before inhalation of plasminogen. Column B: CT images of the chest of the corresponding patients after inhalation of plasminogen. C: 5 patients are subjected to chest CT review 5 days after B-ultrasound examination. Black arrows and boxes indicate anomalies. After administration of human plasminogen for 2-3 times, the number, extent and density of lung lesions in 5 patients are reduced, or even partially disappeared; patchy or punctate "ground glass" shadows are significantly reduced or absorbed. It indicates that aerosol inhalation of human plasminogen can rapidly improve lung injury caused by COVID-19 infection.

Fig. 9 shows the result of heart rate monitoring before and after inhalation of plasminogen in common, severe and critical COVID-19 patients. Statistical analysis shows that after the administration of plasminogen, all the mean heart rates of the common, severe and critical patients have a decreasing trend, and there is a statistically difference in comparison of the heart rates of the common patients before and after the administration ($p < 0.05$).

DETAILED DESCRIPTION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and vascular endothelial cells, and may directly activate plasminogen without the need for fibrin as a cofactor. Plasminogen (PLG) is synthesized by liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and II, etc.; converting plasminogen into plasmin; hydrolyzing complement, etc. Fibrinolysis inhibitors: including plasminogen activator inhibitor (PAI) and $\alpha 2$ antiplasmin ($\alpha 2$-AP). PAI mainly has two forms, PAI-1 and PAI-2, which may specifically bind to t-PA in a ratio of 1:1, thereby inactivating it and activating PLG at the same time. $\alpha 2$-AP is synthesized by liver, and binds to PL in a ratio of 1:1 to form a complex to inhibit the activity of PL; FXIII makes $\alpha 2$-AP covalently bound to fibrin, reducing the sensitivity of fibrin to PL. Substances that inhibit the activity of the fibrinolytic system in vivo: PAI-1, complement C1 inhibitor; $\alpha 2$ antiplasmin; $\alpha 2$ macroglobulin.

The term "component of plasminogen activation pathway" according to the present application encompasses:

1. plasminogen, Lys-plasminogen, Glu-plasminogen, micro-plasminogen, delta-plasminogen; variants or analogs thereof;
2. plasmin and a variant or an analog thereof; and
3. plasminogen activators, such as tPA and uPA, and tPA or uPA variants and analogs comprising one or more domains of tPA or uPA, such as one or more kringle domains and proteolytic domains.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the application encompasses a protein having at least 75%, 80%, 85%, 90%, 95%, 96%, 97%, 98% or 99% sequence identity with SEQ ID NO: 2, 6, 8, 10 or 12, and still retaining plasminogen activity. For example, a "plasminogen variant" according to the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining plasminogen activity. Particularly, the plasminogen variants according to the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid.

The plasminogen according to the present application may be a human plasminogen ortholog from a primate or rodent, or a variant thereof still retaining plasminogen activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle domains and proteolytic domains), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether a "variant" or "analog" of the above plasminogen, plasmin, tPA or uPA respectively has the activity of plasminogen, plasmin, tPA or uPA, or whether the "variant" or "analog" provides substantially similar effect to plasminogen, plasmin, tPA or uPA, may be detected by methods known in the art, for example, it is measured by the level of activated plasmin activity based on enzymography, ELISA (enzyme-linked immunosorbent assay), and FACS (fluorescence-activated cell sorting method), for example, it is detected by referring to a method selected from the following documents: Ny, A., Leonardsson, G., Hagglund, A.C, Hagglof, P., Ploplis, V.A., Carmeliet, P. and Ny, T. (1999). Ovulation in plasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5): 1625-33; Gravanis I, Tsirka SE (February 2008). "Tissue-type plasminogen activator as a therapeutic target in stroke". Expert Opinion on Therapeutic Targets. 12(2): 159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" according to the present application is a plasminogen selected from the group consisting of: Glu-plasminogen, Lys-plasminogen, mini-plasminogen, micro-plasminogen, delta-plasminogen, or variants thereof retaining plasminogen activity. In some embodiments, the plasminogen is natural or synthetic human plasminogen, or a conservative mutant variant or fragment thereof still retaining plasminogen activity. In some embodiments, the plasminogen is a human plasminogen ortholog from a primate or rodent or a conservative mutant variant or fragment thereof still retaining plasminogen activity. In some embodiments, the amino acid sequence of the plasminogen is represented by SEQ ID NO: 2, 6, 8, 10 or 12. In some embodiments, the plasminogen is a human natural plasminogen. In some embodiments, the plasminogen is a human natural plasminogen represented by SEQ ID NO: 2.

"A compound capable of directly activating plasminogen, or indirectly activating plasminogen by activating an upstream component of plasminogen activation pathway", refers to any compound capable of directly activating plasminogen, or indirectly activating plasminogen by activating an upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" according to the present application is a compound that antagonizes, weakens, blocks, or prevents the action of a fibrinolysis inhibitor. Such fibrinolysis inhibitors are e.g., PAI-1, complement C1 inhibitor, α2 antiplasmin, and α2 macroglobulin. Such an antagonist is: e.g., an antibody of PAI-1, complement C1 inhibitor, α2 antiplasmin, or α2 macroglobulin; or an antisense RNA or small RNA blocking or downregulating the expression of such as PAI-1, complement C1 inhibitor, α2 antiplasmin or α2 macroglobulin; or a compound occupying the binding site of PAI-1, complement C1 inhibitor, α2 antiplasmin, or α2 macroglobulin but without the function of PAI-1, complement C1 inhibitor, α2 antiplasmin, or α2 macroglobulin; or a compound blocking the binding domains and/or active domains of PAI-1, complement C1 inhibitor, α2 antiplasmin, or α2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Plasminogen is a single-chain glycoprotein consisting of 791 amino acids with a molecular weight of approximately 92 kDa. Plasminogen is mainly synthesized in liver, and is abundantly present in the extracellular fluid. The content of plasminogen in plasma is approximately 2 μM. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of a two-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor α2-AP. A recently found plasminogen is a 38 kDa fragment, including kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM remodeling. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. In vitro, plasmin also hydrolyzes components of the complement system, and releases chemotactic complement fragments.

"Plasmin" is a very important enzyme present in blood that hydrolyzes fibrin clots into fibrin degradation products and D-dimers.

"Plasminogen" is the zymogen form of plasmin. According to the sequence in swiss prot, it consists of 810 amino acids calculated by the natural human plasminogen amino acid sequence (SEQ ID NO: 4) containing the signal peptide, and the molecular weight is about 90kD, and it is a glycoprotein mainly synthesized in liver and capable of circulating in blood, the cDNA sequence encoding this amino acid sequence is represented by SEQ ID NO: 3. Full-length plasminogen contains seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a human natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. In vivo, there is also a Lys-plasminogen produced by the hydrolysis of the peptide bond between amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and a serine protease domain (also known as a protease domain (PD)). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), the amino acid sequence of the mini-plasminogen is represented by SEQ ID NO: 10, and the cDNA sequence encoding this amino acid sequence is represented by SEQ ID NO: 9. While micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); in the present patent application, the sequence of micro-plasminogen refers to the patent document CN102154253A, the amino acid sequence is represented by SEQ ID NO: 12, and the cDNA sequence encoding this amino acid sequence is represented by SEQ ID NO: 11.

The structure of the full-length plasminogen is also described in the article by Aisina et al. (Aisina RB, Mukhametova L I. Structure and function of plasminogen/plasmin system [J]. Russian Journal of Bioorganic Chemistry, 2014, 40(6):590-605). In this article, Aisina et al. describe that plasminogen comprises Kringle 1, 2, 3, 4, 5 domains and a serine protease domain (also called protease domain (PD))(i.e., lysine binding activity), wherein Kringles are responsible for binding of plasminogen to low or high molecular weight ligand, so that plasminogen transforms into a more open conformation that is more readily activated; the protease domain (PD) is residues Val562-Asn791; the Arg561-Val562 activating bond of plasminogen is specifically cleaved by tPA and uPA, thereby allowing plasminogen to change into plasmin; thus the protease domain (PD) is a region conferring the proteolytic activity of plasminogen.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen according to the present application are applicable to plasmin, thus the technical solutions described in the present application encompass plasminogen and plasmin. During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

An "active fragment of plasminogen" refers to a fragment having the activity of binding to a lysine in the target sequence of a substrate (lysine-binding activity), or exerting the activity of a proteolytic function (proteolytic activity), or having a combination of proteolytic activity and lysine-binding activity. The technical solutions related to plasminogen according to the present application encompass the technical solutions of replacing plasminogen with an active fragment of plasminogen. In some embodiments, the active fragment of plasminogen according to the present application comprises or consists of a serine protease domain of plasminogen, preferably the active fragment of plasminogen according to the present application comprises or consists of SEQ ID NO: 14, or an amino acid sequence having at least 80%, 90%, 95%, 96%, 97%, 98%, 99% identity with SEQ ID NO: 14. In some embodiments, the active fragment of plasminogen according to the present application comprises or consists of one or more regions selected from the group consisting of: Kringle 1, Kringle 2, Kringle 3, Kringle 4, and Kringle 5. In some embodiments, the plasminogen according to the present application comprises a protein comprising the active fragment of plasminogen described above.

At present, the methods for measuring plasminogen and its activity in blood comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is proportional to plasminogen activity. In addition, the plasminogen activity in blood may also be detected by immunochemical method, gel electrophoresis, immunoturbidimetry, and radioimmunoassay.

"Ortholog or orthologs" refer to homologs between different species, including both protein homologs and DNA homologs, also known as orthologs and vertical homologs; particularly it refers to proteins or genes evolved from the same ancestral gene in different species. The plasminogen according to the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having not less than 60%, preferably not less than 75%, more preferably not less than 85%, or even most preferably not less than 90% amino acid identity determined by BLAST or FASTA algorithm, and having the same or substantially similar properties or functions as the natural or parent protein or enzyme.

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or non-reducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:

$$\text{Fraction X/Y times 100};$$

wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

As used herein, the terms "treatment/treating" refer to obtaining a desired pharmacological and/or physiological effect. The effect may be complete or partial prevention of the occurrence, or onset of the disease or symptoms thereof, partial or complete alleviation of the disease and/or symptoms thereof, and/or partial or complete cure of the disease and/or symptoms thereof; and includes: (a) preventing the occurrence or onset of the disease in a subject, who may have predisposition of the disease, but is not yet diagnosed as having the disease; (b) inhibiting the disease, i.e., blocking its development; and (c) alleviating the disease and/or symptoms thereof, i.e., causing regression or elimination of the disease and/or symptoms thereof.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of a component of plasminogen activation pathway or a related compound thereof (e.g., plasminogen) sufficient to prevent and/or treat a disease when administrated to a mammal or other subject for treating the disease. A "therapeutically effective amount" will vary depending on the component of plasminogen activation pathway or a related compound thereof (e.g., plasminogen) in use, the severity of the disease and/or symptoms thereof in the subject to be treated, as well as the age, weight, and the like.

**Preparation of the Plasminogen According to the Present Application**

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen according to the present application may be produced by standard recombinant methods. For example, a nucleic acid encoding plasminogen is inserted into an expression vector to be operably linked to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector is typically replicated in a host organism as an episome or as an integrated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a subject antibody-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., S. *cerevisiae*) and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization.

In addition to microorganisms, mammalian cells (e.g., mammalian cells grown in in vitro cell culture) may also be used to express and produce the anti-Tau antibodies of the application (e.g., polynucleotides encoding the subject anti-Tau antibodies). See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the target product, and the like.

**Medicament Formulation**

A therapeutic formulation may be prepared by mixing the component of plasminogen activation pathway or a related compound thereof (e.g., plasminogen) of desired purity with an optional pharmaceutical carrier, excipient, or stabilizer (Remington's Pharmaceutical Sciences, 16th edition, Osol, A. ed. (1980)), to form a lyophilized formulation or an aqueous solution. An acceptable carrier, excipient, or stabilizer is non-toxic to a recipient at the employed dosage and concentration, including buffers such as phosphate, citrate and other organic acids; antioxidants such as ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG). Preferred lyophilized anti-VEGF antibody formulation is described in WO 97/04801, which is incorporated herein by reference.

The formulations according to the present application may also contain more than one active compound as required for the particular condition to be treated, preferably those compounds are complementary in activity and do not have side effects with each other.

The plasminogen according to the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The component of plasminogen activation pathway or a related compound thereof (e.g., plasminogen) according to the present application for in vivo administration must be sterile. This may be easily achieved by filtration through sterilizing filters before or after lyophilization and reformulation.

The component of plasminogen activation pathway or a related compound thereof (e.g., plasminogen) according to the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot™ (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for more than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix composition.

## Administration and Dosage

Administration of the pharmaceutical composition according to the present application may be accomplished by different means, e.g., nasal inhalation, aerosol inhalation, nasal or eye drop, intravenous administration, intraperitoneal administration, subcutaneous administration, intracranial administration, intrathecal administration, intraarterial administration (e.g., via the carotid artery), intramuscular administration, and rectal administration.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other concomitantly administrated medicaments. The dosage range of the pharmaceutical composition comprising the plasminogen according to the present application may be, for example, about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 0.01-100 mg/kg on consecutive days. Real-time evaluation of therapeutic efficacy and safety is required during the administration of the medicament of the present application.

## Product or Kit

One embodiment of the present application relates to a product or kit comprising a component of plasminogen activation pathway or a related compound thereof (e.g., plasminogen). The product preferably comprises a container, a label or package insert. Suitable containers are bottles, vials, syringes, etc. The container may be made of various materials such as glass or plastic. The container contains a composition which is effective for treatment of the disease or condition according to the present application and has a sterile access port (e.g., the container may be an intravenous solution pack or vial containing a stopper penetrable by a hypodermic needle). At least one active agent in the composition is a component of plasminogen activation pathway or a related compound thereof (e.g., plasminogen). The label on or attached to the container indicates that the composition is used for treatment of the diseases mentioned in the present application. The product may further comprise a second container containing a pharmaceutically acceptable buffer, such as phosphate buffered saline, Ringer's solution, and glucose solution. It may further contain other materials required from a commercial and user standpoint, including other buffers, diluents, filters, needles and syringes. In addition, the product comprises a package insert with instructions for use, including, for example, instructing the user of the composition to administrate the composition comprising a component of plasminogen activation pathway or a related compound thereof (e.g., plasminogen) to the patient along with other medicaments for treatment of concomitant diseases.

EXAMPLES

Human plasminogen used in the following examples is derived from plasma of a human donor, based on methods described in: Kenneth C Robbins, Louis Summaria, David Elwyn et al. Further Studies on the Purification and Characterization of Human Plasminogen and Plasmin. Journal of Biological Chemistry, 1965, 240(1): 541-550; Summaria L, Spitz F, Arzadon L et al. Isolation and characterization of the affinity chromatography forms of human Glu-and Lys-plasminogens and plasmins. J Biol Chem. 1976 Jun 25;251(12):3693-9; HAGAN JJ, ABLONDI FB, DE RENZO EC. Purification and biochemical properties of human plasminogen. J Biol Chem. 1960 Apr; 235:1005-10, with process optimization, being purified from plasma of a human donor, with >98% human Lys-plasminogen and Glu-plasminogen.

**Example 1: Plasminogen Reduces the Level of Pulmonary Fibrosis in the Model Mice of Monocrotaline-induced Pulmonary Hypertension**

Twelve 12-week-old C57 male mice are weighed, and their blood pressures are measured. According to their blood pressures, they are randomly divided into 2 groups; 4 mice in the blank control group, and 8 mice in the model group. The mice in the blank control group are injected with 100 $\mu$l of normal saline through the tail vein, and the mice in the model group are injected with 60 mg/kg/mouse of monocrotaline at a single injection through the tail vein, constructing the model for 3 days, and normally feeding the mice [3-4]. The blood pressure is measured 3 days later, and the mice in the model group are randomly divided into two groups according to the blood pressure; with 4 mice in each of the vehicle PBS control group and the plasminogen group. The mice in the plasminogen group are given plasminogen by tail vein injection at 1 mg/0.1 ml/mouse/day, and the mice in the vehicle PBS control group are given the same volume of PBS solution by tail vein injection for 28 consecutive days; the mice in the blank control group are not treated with plasminogen. The start of modeling and administration of plasminogen is set as day 1, and on day 29 the mice are sacrificed to collect the lungs and fix in 4% paraformaldehyde fix solution for 24 hours. The fixed lung tissues are dehydrated with ethanol gradient and cleared with xylene before being embedded in paraffin. The thickness of the tissue section is 3 $\mu$m. After the sections are dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200$\times$ optical microscope.
Monocrotaline is a pyrrolizidine alkaloid, which is converted by P450 mono-oxygenase in the liver and reaches the lungs through the blood circulation, causing irreversible damage to the pulmonary blood vessels. Pulmonary vascular endothelial cells are considered to be the target cells of monocrotaline, and endothelial cell injury plays a key role in the process of pulmonary vascular remodeling.
The results show that, there is basically no collagen deposition in the lungs of the mice in the blank control group (Fig. 1A), and the collagen deposition (marked by arrows) in the lung tissues of the mice in the plasminogen group (Fig. 1C) is significantly less than that in the control group (Fig. 1B). It indicates that plasminogen can significantly reduce the pulmonary fibrosis in the model mice of monocrotaline-induced pulmonary hypertension.

**Example 2: Plasminogen Reduces Pulmonary Fibrosis in Mice with Systemic Sclerosis**

Seventeen 12-week-old C57 male mice are randomly divided into two groups; with 11 in the vehicle PBS control group, and 6 in the plasminogen group. The day of the beginning of the experiment is recorded as day 0, and the mice are weighed to divide into groups. On day 1, the administration is started for modeling, the two groups of mice are injected subcutaneously with 0.1mg/0.1ml/mouse/day bleomycin to induce systemic sclerosis [5], and the administration of plasminogen or PBS is started and continued to construct the model for 21 days. The mice in the plasminogen group are injected with plasminogen at 1 mg/0.1 ml/mouse/day through the tail vein, and the mice in the vehicle PBS control group are given the same volume of PBS in the same way. On day 22, the mice are sacrificed to collect lung tissues and fix them in 4% paraformaldehyde fix solution for 24 hours. The fixed lung tissues are dehydrated with ethanol gradient and cleared with xylene before being embedded in paraffin. The thickness of the tissue section is 3 $\mu$m. After the sections are dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200$\times$ optical microscope.

The study finds that in the model mice of bleomycin-induced systemic sclerosis, the degree of collagen fibrosis in the PBS group (Fig. 2A) is higher than that in the plasminogen group (Fig. 2B). The morphology of pulmonary alveolar wall of the mice in the plasminogen group is close to the normal level, the inflammatory cells are significantly reduced, and the degree of fibrosis is significantly lower than that in the vehicle PBS group, and the statistical difference is significant (Fig. 2C). This indicates that plasminogen can effectively reduce pulmonary fibrosis in the mice with systemic sclerosis induced by bleomycin.

**Example 3: Plasminogen Reduces Pulmonary Fibrosis in the Mice of Paraquat Poisoning**

Twelve 9-10-week-old C57 mice are randomly divided into two groups; with 6 mice in each of the vehicle PBS control group and the plasminogen group. The two groups of mice are administrated with plasminogen immediately after being given a single intraperitoneal injection of 15 mg/kg body weight of paraquat solution, recording as day 1 [6]. The mice in the plasminogen group are injected with plasminogen through tail vein at 1 mg/0.1 mL/mouse/day, and the mice in the vehicle PBS control group are given the same volume of PBS in the same way. The dosing cycle is 14 days, on day 15 the mice are sacrificed to collect the lung tissues and fix them in 4% neutral formalin fix solution for 24 hours. The fixed lung tissues are dehydrated with ethanol gradient and cleared with xylene before being embedded in paraffin. The thickness of the tissue section is 3 $\mu$m. After the sections are dewaxed to water, washing once with water, staining with 0.1% Sirius red saturated picric acid for 60 min, rinsing with running water; staining with hematoxylin for 1 min, rinsing with running water, differentiating with 1% hydrochloric acid in ethanol, and returning to blue in ammonia solution, rinsing with running water; then drying and sealing, and finally observing under a 200$\times$ optical microscope.

Paraquat is a fast-killing herbicide that is extremely toxic to humans, and can damage the lungs, liver, and kidneys when it enters the human body. Lung injury manifests as early alveolar epithelial cell damage, intraalveolar hemorrhage and edema and other symptoms; at the late stage manifesting as intraalveolar and pulmonary interstitial fibrosis [6]. Currently, there is no treatment for paraquat poisoning, and the mortality rate is almost 100%.

The results of Sirius red staining show that, the collagen fibers deposition in the lungs of the mice in the plasminogen group (Fig. 3B) is significantly less than that in the vehicle PBS control group (Fig. 3A). It indicates that plasminogen can reduce pulmonary fibrosis caused by paraquat poisoning.

**Example 4: Plasminogen Reduces Collagen Deposition in Lung Tissues of the Mice of Pneumonia Model**

Eighteen 6-8 week-old C57 mice are randomly divided into three groups according to body weight, i.e., blank control group, vehicle group, and plasminogen group; with 6 mice in each group. After the mice in the vehicle group and the plasminogen group are anesthetized with 2% isoflurane, 1.5 mg/ml of bacterial lipopolysaccharide (LPS) solution (purchased from Beijing Solarbio Technology Co., Ltd., Cat. No.: L8880) is instilled in the trachea according to 3 mg/kg body weight to construct a pneumonia model [7]. Two hours after administration of LPS, the mice in the plasminogen group are injected with plasminogen at 1 mg/0.1 ml/mouse/day through the tail vein, and the mice in the vehicle group are injected with the same volume of vehicle through the tail vein, the treatment is lasted for 14 consecutive days. On day 15, the mice are sacrificed to collect the lung tissues and fix them in 4% neutral formalin fix solution for 24 hours. The fixed lung tissues are dehydrated with ethanol gradient and cleared with xylene before being embedded in paraffin. The thickness of the tissue section is 3 $\mu$m. After the sections are dewaxed to water, washing once with water, staining with 0.1% Sirius red saturated picric acid for 60 min, rinsing with running water; staining with hematoxylin for 1 min, rinsing with running water, differentiating with 1% hydrochloric acid in ethanol, and returning to blue in ammonia solution, rinsing with running water; then drying and sealing, and finally observing under a 200$\times$ optical microscope.

The results show that, the lung tissues of the blank control group (Fig. 4A) have a certain amount of collagen deposition (marked by arrows), the collagen deposition in the lung tissues of the mice in the vehicle group (Fig. 4B) is significantly increased, and the collagen deposition in the lung tissues of the mice in the plasminogen group (Fig. 4C) is significantly less than that in the vehicle group, and the statistical difference is significant (* means P<0.05) (Fig. 4D). It indicates that plasminogen can reduce the collagen deposition in the lungs of the mice with pneumonia, and improve the pulmonary fibrosis caused by pneumonia.

**Example 5: Administration of Plasminogen in Advance Reduces Collagen Deposition in Lung Tissues of the Model Mice of Pneumonia**

Eighteen 6-8-week-old C57 mice are randomly divided into three groups according to body weight, i.e., blank control group, vehicle group, and plasminogen group; with 6 mice in each group. The mice in the plasminogen group are injected with plasminogen at 1 mg/0.1 ml/mouse/day through the tail vein, and the mice in the vehicle group are injected with the same volume of vehicle through the tail vein, the treatment is lasted for 3 consecutive days. On day 4, the mice in the vehicle group and the plasminogen group are anesthetized with 2% isoflurane, and 1.5 mg/ml of bacterial lipopolysaccharide (LPS) solution (purchased from Beijing Solarbio Technology Co., Ltd., Cat. No.: L8880) is instilled in the trachea according to 3 mg/kg body weight to construct a pneumonia model [7]. On day 9, the mice are sacrificed to collect the lung tissues and fix them in 4% neutral formalin fix solution for 24 hours. The fixed lung tissues are dehydrated with ethanol gradient and cleared with xylene before being embedded in paraffin. The thickness of the tissue section is 3 $\mu$m. After the sections are dewaxed to water, washing once with water, staining with 0.1% Sirius red saturated picric acid for 60 min, rinsing with running water; staining with hematoxylin for 1 min, rinsing with running water, differentiating with 1% hydrochloric acid in ethanol, and returning to blue in ammonia solution, rinsing with running water; then drying and sealing, and finally observing under a 200$\times$ optical microscope.

The results show that, the lung tissues of the blank control group (Fig. 5A) have a certain amount of collagen deposition (marked by arrows), the collagen deposition in the lung tissues of the vehicle group (Fig. 5B) is significantly increased, and the collagen deposition in the lung tissues of the mice in the plasminogen group (Fig. 5C) is significantly less than that in the vehicle group. It indicates that the administration of plasminogen in advance can reduce the collagen deposition in the lungs of the mice with pneumonia, and improve the pulmonary fibrosis caused by pneumonia.

**Example 6: Plasminogen Reduces the Level of Total Protein in Lung Lavage Fluid of the Model Mice of Pneumonia**

Eighteen 6-8-week-old C57 mice are randomly divided into three groups according to body weight, i.e., blank control group, vehicle group, and plasminogen group; with 6 mice in each group. After the mice in the vehicle group and the plasminogen group are anesthetized with 2% isoflurane, 1.5 mg/ml of bacterial lipopolysaccharide (LPS) solution (purchased from Beijing Soloarbio Technology Co., Ltd., Catalog number: L8880) is instilled in the trachea according to 3 mg/kg body weight to construct a pneumonia model. Two hours after administration of LPS, the mice in the plasminogen group are injected with plasminogen at 1 mg/0.1 ml/mouse/day through the tail vein, and the mice in the vehicle group are injected with the same volume of vehicle through the tail vein, the treatment is lasted for 7 consecutive days. On day 8, all mice are sacrificed, and the lungs are dissected and weighed. The right lung bronchus is subjected to lavage with PBS twice continuously in a total of 0.7 ml. The liquid is collected and centrifuged at 700g at 4°C for 10 min, collecting the supernatant to detect the total protein by Bicinchoninic acid (BCA) assay.

The results show that, there is a certain amount of total protein in the lung lavage fluid of the blank control group, and the total protein level in the lung lavage fluid of the vehicle group is significantly higher than that of the blank control group (*** means P<0.001), while the total protein level in the lung lavage fluid of the plasminogen group is significantly lower than that of the vehicle group, and the statistical difference is close to significance (P=0.052) (Fig. 6). It indicates that plasminogen can reduce the total protein level in lung lavage fluid of the model mice of pneumonia.

**Example 7: Plasminogen Reduces Deposition of Pulmonary Fibrin in the Mice with Pneumonia**

Twelve 6-8-week-old C57 mice are randomly divided into two groups according to body weight, i.e., the vehicle group and the plasminogen group; with 6 mice in each group. After the mice in the vehicle group and the plasminogen group are anesthetized with 2% isoflurane, 1.5 mg/ml of bacterial lipopolysaccharide (LPS) solution (purchased from Beijing Soloarbio Technology Co., Ltd., Catalog number: L8880) is instilled in the trachea according to 3 mg/kg body weight to construct a pneumonia model [7]. Two hours after administration of LPS, the mice in the plasminogen group are injected with plasminogen at 1 mg/0.1 ml/mouse/day through the tail vein, and the mice in the vehicle group are injected with the same volume of vehicle through the tail vein, the treatment is lasted for 3 consecutive days. On day 4, the mice are sacrificed to collect the left lung and fix it in 4% neutral formalin fix solution for 24 hours. The fixed lung tissue samples are dehydrated with ethanol gradient and cleared with xylene before being embedded in paraffin. The thickness of the tissue section is 3 $\mu$m. After the tissue sections are dewaxed and rehydrated, washing them once with water, repairing with citric acid for 30 min, then cooling at room temperature for 10 min and rinsing gently with water. The tissue sections are incubated with 3% hydrogen peroxide for 15 min, circling the tissues with a PAP pen; blocking with 10% goat serum (Vector laboratories, Inc., USA) for 1 hour, the goat serum is discarded when the time is up. The tissue sections are incubated in rabbit-derived anti-Fibrin antibody (Abeam) overnight at 4°C, washing twice with PBS for 5 min each time; then incubating in goat anti-rabbit IgG (HRP) antibody (Abeam) secondary antibody at room temperature for 1 hour, and washing twice with PBS for 5 min each time. The color is developed according to the DAB kit (Vector laboratories, Inc., USA), after washing with water for 3 times, counterstaining with hematoxylin for 30 seconds, returning to blue under running water for 5 min, and then washing once with PBS. After gradient dehydration, the tissue sections are subjected to being cleared and sealed, finally observing under a 200× optical microscope.

Fibrin is the main component of the hyaline membrane of the lung in acute respiratory distress. The hyaline membrane hinders the gas exchange function of the alveoli, resulting in decreased blood oxygen saturation and dyspnea [8].

The results show that, the level of fibrin deposition in the lung tissues of the mice in the vehicle group (Fig. 7A) is significantly higher than that in the plasminogen group (Fig. 7B). It indicates that plasminogen can reduce fibrin deposition of lung in the model mice of pneumonia.

The following examples further illustrate the practice of the application, but are not intended to limit the application.

All the following patients signed the informed consent forms and are voluntarily administrated plasminogen, and are approved by the hospital ethics committee.

The usage and dosage of plasminogen are adjusted according to the severity and course of the disease. The main modes of administration are aerosol inhalation and intravenous injection. The concentrations of human plasminogen purified from the above human donor blood, used in aerosol inhalation and intravenous injection are all 5 mg/ml, and normal saline is used as a vehicle.

**Example 8: Patient with Pneumonia and Pulmonary Fibrosis**

**The patient:** a 77-year-old male, he has sequelae of cerebral infarction and pulmonary fibrosis. Before administration of plasminogen, he manifested as pneumonia, and the sputum was thick, yellow, viscous, and difficult to cough up; sputum suction was required, 25-26 times a day (24 hours), and a large amount of sputum was sucked out each time. He needed oxygen inhalation all the time (he almost could not live without oxygen inhalation), and the breath sounds were slightly rough. Laryngeal trachea incision was performed (being assisted with metal sleeve). He has been bedridden for nearly half a year, with poor sleep and poor mental state.

**Therapeutic regimen:** in the first 9 days, plasminogen is administrated by aerosol inhalation, 10 mg/time, 3 times/day; on day 10, aerosol inhalation, 10 mg/time, 2 times/day; on day 11, no administration of plasminogen; on day 12, aerosol inhalation once at a dose of 10mg; on day 13, aerosol inhalation once at a dose of 10mg, in combination with 10mg of intravenous injection; on day 14, intravenous injection at a dose of 15mg.

**Therapeutic effects:** on day 1 of administration of plasminogen: the symptoms are alleviated, manifesting as strong expectoration, spontaneous discharge from the trachea incision, and the times of sputum suctions reduce to 8 times (from 8:00 a.m. to 8:00 p.m.). On day 2 of administration of plasminogen: at the first time he can sit without oxygen inhalation for 20 min by being helped with armrest, the blood oxygen saturation is 94%, and the heart rate is 65; at the second time he can sit without oxygen inhalation for more than 10 min, the blood oxygen saturation is 98%, and the heart rate is 75; at the third time he can sit without oxygen inhalation for 20 min by being helped with armrest, the blood oxygen saturation is 94-98%, and the heart rate is about 78. The expectoration is stronger than the day before, and the suction is performed 6 times (from 8:00 a.m. to 8:00 p.m.). On day 14 of administration of plasminogen: the patient actively requests to give up oxygen inhalation; after giving up the oxygen inhalation, the blood oxygen saturation is 97%, and about 94% when falling asleep; and the time without oxygen inhalation in a whole day can reach more than 12 hours, basically he can live without oxygen inhalation; the sputum of the patient is changed from the yellow, thick, viscous state to white, thin and foamy state; and the patient changes from mainly excreting the sputum with the help of a sputum suction machine to excreting most of the sputum spontaneously.

The above results show that, plasminogen can effectively improve the symptoms of pneumonia in a patient, including strong expectoration, spontaneous expectoration, reduction of sputum suction times, prolonged time without oxygen inhalation, improvement of blood oxygen saturation, and improvement of sputum properties.

**Example 9: Patient with Pulmonary Fibrosis and Asthma**

**The patient:** an 83-year-old female, she suffered from pulmonary tuberculosis when she was young. About 20 years ago, she was diagnosed with pulmonary fibrosis and asthma, which gradually worsened. Main manifestations: poor lung function, she basically could only support activities on bed, and could not get out of bed.

**Therapeutic regimen:** on day 1, administration of plasminogen is performed by aerosol inhalation, 10 mg/time, once a day; on day 2, aerosol inhalation, 10 mg/time, twice a day; on day 3, aerosol inhalation, 10 mg/time, 3 times a day.

**Therapeutic effects:** after administration of plasminogen, the patient can breathe smoothly, and she can support more activities after getting out of bed.

The above results show that, plasminogen can improve lung function in a patient with pulmonary fibrosis and asthma.

**Example 10: Asthma Patient**

**The patient:** a 55-year-old male, he has stable vital signs and clear consciousness. Chief complaint: no history of hypertension, heart disease, and diabetes, etc. The patient had been taking trimetazidine and bayaspirin, ginkgo leaf capsules and deep-sea fish oil for a long time, and suffered from cerebral infarction 3 years ago. The patient complained of poor sleep quality, no other discomfort symptoms. The patient was observed for asthma after exercise.

**Therapeutic regimen:** the treatment consists of 14 doses of plasminogen over 22 days. In the first 6 days, plasminogen is administered once a day at a dose of 150 mg intravenous bolus injection each time; in the next 16 days, a dose of 150 mg intravenous bolus injection is administered once every two days.

**Therapeutic effects:** the treatment effects are assessed by scoring the patient's overall condition and post-exercise asthma symptoms. On day 1, the score of the patient's overall condition and post-exercise asthma symptoms without administration of plasminogen is set as 10 points, and after the administration the score of the patient's overall condition and post-exercise asthma symptoms of yesterday is set as 10 points. Particularly, 10 points is the most severe, and 1 point is the lightest.

On day 4 of administration of plasminogen, the overall condition score is 8 points, the post-exercise asthma symptom score is 8 points, the patient's mental state is better, and the sleep quality is improved. With the prolongation of administration time, various symptoms of the patient are further improved. On day 22 of administration of plasminogen, the overall condition score is 5 points, the post-exercise asthma symptom score is 3 points, the patient has no discomfort, the sleep quality is further improved, and the appetite is improved.

It can be seen from the above that, plasminogen can improve the symptoms of asthma patients, including general condition, post-exercise asthma symptoms, mental state, sleep quality, appetite, etc.

**Example 11: Non-5q SMA Patient**

**The patient:** a 40-month-old female (3 years and 4 months), she was diagnosed with non-5q type SMA (spinal muscular atrophy). She developed SMA when she was 6 months old, and was diagnosed with SMA at 1.5 years old (18 months old). She had shallow breathing due to lung infection and difficulty in expelling sputum to block the respiratory tract. After intermittent use of the breathing machine, she gradually failed to take off the breathing machine, and continuously used the breathing machine for about 1.5 years. She lost language function and was unable to move, the muscle strength was basically grade 0. Symptoms: breathing machine is used until now, daily use of sputum excretion device, expectoration machine, sputum suction device, oxygen inhalation, aerosol inhalation (twice a day), and nasal feeding. Parameters of the breathing machine in use: pressure 20 cm $H_2O$; tidal volume around 120 ml; almost no spontaneous breathing, oxygen flow 1-1.5 ml, sputum suction 15-20 times a day, irregular sputum suction at night (tracheal mucosa damage and bleeding had ever been caused by sputum suction). The blood oxygen detector shows that the blood oxygen saturation is above 95% under the condition of oxygen use (oxygen supply cannot be stopped), and the heart rate was 140 beats/min. The body temperature was above 37.5°C for a long time.

**Therapeutic regimen:**

The first course of treatment (2 weeks): aerosol inhalation, 10 mg/time, 3 times/day; in combination with 50-100 mg intravenous injection, once every three days.

After completion of the first course of treatment, an interval of 57 days is followed to start the second course of treatment.

The second to the fourth courses of treatment (there is a 2-week interval between adjacent courses): intravenous injection, 1 time/3 days, with a dose of 150-250 mg.

**Therapeutic effects:**

The first course of treatment: the parameters of the breathing machine remain unchanged, and the spontaneous breathing increases to about 20-30% (about 2-3 spontaneous breaths occur in 10 breaths). When the oxygen flow remains unchanged, after administration of plasminogen the blood oxygen saturation can reach 98-99%.

The second course of treatment: after 15 days of hospitalization, tracheotomy is performed, and the patient is discharged home for nursing care. The amount of sputum is too much to overflow from the trachea all the time, and it needs to be cleaned up in time by suction; the pressure of the breathing machine is reduced from 18 when discharged from the hospital to 16, the tidal volume is 130-140, and the spontaneous breathing recovers well; oxygen supply can be turned off, basically no oxygen is needed at night; the blood oxygen saturation is above 95%, heart rate is about 120 beats/min, sometimes better, and body temperature returns to normal.

The third course of treatment: the pressure of the breathing machine can be adjusted to 15 or 16, the tidal volume is 140-150, the trachea is more open, the patient maintains spontaneous breathing all the time, the times of oxygen inhalations is few, and oxygen inhalation is occasionally performed during sputum suction. Normal heart rate is 120 beats/min, after administration of plasminogen the oxygen supply may be stopped for 4-5 h, the heart rate is 100 beats/min, and the blood oxygen saturation is 99%. The patient's sputum volume is reduced, the trachea incision is basically free of sputum, and sputum suction is performed 5-6 times a day.

The fourth course of treatment: the parameters of the breathing machine and spontaneous breathing are the same as those in the third course of treatment, the blood oxygen saturation is 97-99% everyday, basically no oxygen supply is needed, and the times of sputum suctions is reduced, 2-3 times a day, and occasionally 5-6 times. Body temperature is normal.

The above results show that, plasminogen can improve lung function in SMA patients, including improvement of blood oxygen saturation; improvement of spontaneous breathing function, trachea patency, no oxygen inhalation or occasional oxygen inhalation; reduction of sputum volume.

**Example 12: Type I SMA Patient**

**The patient:** an 18-month-old male, was diagnosed with type I SMA at 6 months. At the time of diagnosis, doctors informed that the average survival period of a patient with this type of disease is 2 years, and no treatment measure was taken by the family members. Pulmonary function symptoms: the blood oxygen is monitored for 24 hours in a whole day, blood oxygen saturation is 92-97%; chest heaves gently during breathing, and breathing is weak during sleep; sputum sounds, and the patient is unable to expectorate sputum spontaneously. Crying is weak with poor spirit.

**Therapeutic regimen:** aerosol inhalation (2-3 times/day) + intravenous injection (1 time/3 days), the treatment cycle is 5 courses of treatment (2 weeks as a course of treatment), and there is a 2-week interval between adjacent courses of treatment. The dose of aerosol inhalation is 5-10mg/time; the dose of intravenous injection is 50mg.

**Therapeutic effects:**

The first course of treatment: on day 2 of the treatment, the blood oxygen saturation reaches 97-98%, occasionally 95-96%. Blood oxygen saturation reaches normal, respiratory function improves, sputum expectoration can be achieved with assistance, and sputum volume is reduced.

The second course of treatment: the blood oxygen saturation remains normal, and the breathing is stronger.

The third course of treatment: sputum is excreted after administration of plasminogen by aerosol inhalation, there is a sputum sound in the morning, and the sputum sounds louder after crying, and the sputum can be excreted with assistance.

The fourth to the fifth courses of treatment: the effect is maintained, and the mental state is good, and there are no exacerbation conditions such as pulmonary infection and respiratory failure.

It can be seen from the above that, plasminogen can improve pulmonary function in a patient with type I SMA, including improving respiratory function and increasing blood oxygen saturation. After treatment, the blood oxygen saturation reaches the normal value; sputum can be excreted with assistance; the symptoms of the patient are improved, and there are no exacerbation conditions such as pulmonary infection and respiratory failure.

**Example 13: Patient with Corona Virus Disease 2019 (COVID-19)**

**The patient:** a 48-year-old male, he is diagnosed with severe novel coronavirus pneumonia (2019-nCoV). In the intensive care unit, the breathing machine and monitor have been used, the oxygen concentration is 100%, and the blood oxygen saturation is 80-90%. Vital signs: the heart rate is 92 beats/min, respiration rate is 41 times/min, and blood pressure is 128/84 mmHg.

**Therapeutic regimen:** aerosol inhalation, 10mg/time, two administrations of plasminogen with an interval of 5.5h.

**Therapeutic effects:**

The first administration: before administration of plasminogen, the blood oxygen saturation is 84%, the heart rate is 92 beats/min, the respiration rate is 41 times/min, and the blood pressure is 128/84 mmHg; 1 hour after the administration, the blood oxygen saturation is 90%, the heart rate is 83 beats/min, the respiration rate is 37 times/min, and the blood pressure is 128/84 mmHg.

The second administration: before administration of plasminogen, the blood oxygen saturation is 88%, the heart rate is 81 beats/min, the respiration rate is 39 times/min, and the blood pressure is 120/83 mmHg; 1 hour after the administration, the blood oxygen saturation is 91%, the heart rate is 70 beats/min, the respiration rate is 28/min, and the blood pressure 120/83 mmHg.

There is no adverse reaction before and after the administration, and the patient reports feeling better.

It can be seen from the above that, plasminogen can effectively improve the blood oxygen saturation, slow down the respiratory rate, and improve the lung function in a critical patient with novel coronavirus pneumonia.

**Example 14: COVID-19 Patient**

**The patient:** a 47-year-old male, he is diagnosed with severe novel coronavirus pneumonia (2019-nCoV). In the intensive care unit, the breathing machine and monitor have been used, the oxygen concentration is 100%, and the blood oxygen saturation is 80-90%. Vital signs: the body temperature is 37.5°C, heart rate is 110 beats/min, respiration rate is 37 times/min, and blood pressure is 139/94 mmHg.

**Therapeutic regimen:** aerosol inhalation, 10mg/time, two administrations of plasminogen with an interval of 4 hours and 50 min.

**Therapeutic effects:**

The first administration: before administration of plasminogen, the blood oxygen saturation is 81%; 3 hours after the administration, the blood oxygen saturation is 88-90%.

The second administration: before administration of plasminogen, the blood oxygen saturation is 89-92%, heart rate is 91 beats/min, respiration rate is 31 times/min, and blood pressure is 130/87 mmHg; 1 hour after the administration, the blood oxygen saturation is 91%, heart rate is 89 beats/min, respiration rate is 27 times/min, and blood pressure is 130/87mmHg.

There is no adverse reaction before and after the administration, and the patient reports feeling better.

It can be seen from the above that, plasminogen can effectively increase the blood oxygen saturation, and improve the respiratory rate in a critical patient with novel coronavirus pneumonia.

**Example 15: Plasminogen Reduces Blood Pressure in a Severe COVID-19 Patient**

**The patient:** a 46-year-old male with a blood oxygen saturation of 93% and a respiratory rate of 26 times/min, he is diagnosed with severe COVID-19 according to the clinical classification criteria of the Novel Coronavirus Pneumonia Diagnosis and Treatment Program (Trial Version 6). The patient's blood pressure is 130/80 mmHg (systolic/diastolic) before treatment.

Human plasminogen lyophilized powder is dissolved in sterile water at a concentration of 5 mg/ml, and the solution is administered to the patient after being nebulized by a nebulizer; two administrations every day with a dose of 10mg each time.

After 6 doses of administrations, the patient's blood pressure decreases from the previous 130/80 mmHg to 116/69 mmHg. It indicates that plasminogen can reduce blood pressure in a severe COVID-19 patient.

**Example 16: Plasminogen Reduces Respiratory Rate in a Severe COVID-19 Patient**

**The patient:** a 47-year-old male with a blood oxygen saturation of 93%, he is diagnosed with severe COVID-19 according to the clinical classification criteria of the Novel Coronavirus Pneumonia Diagnosis and Treatment Program (Trial Version 6). The patient's respiratory rate is 45 times/min before treatment.

Human plasminogen lyophilized powder is dissolved in sterile water at a concentration of 5 mg/ml, and the solution is administered to the patient after being nebulized by a nebulizer; two administrations every day with a dose of 10mg each time.

After 2 doses of administrations, the patient's breathing rate decreases from previous 45 times/min to 37 times/min. It indicates that plasminogen can reduce respiratory rate in a severe COVID-19 patient.

**Example 17: Plasminogen Reduces Blood Pressure in a Critical COVID-19 Patient**

**The patient:** a 48-year-old male with a blood oxygen saturation of 79% and a respiratory rate of 41 times/min, he is diagnosed with critical COVID-19 according to the clinical classification criteria of the Novel Coronavirus Pneumonia Diagnosis and Treatment Program (Trial Version 6). The patient's blood pressure is 128/84 mmHg (systolic/diastolic) before treatment.

Human plasminogen lyophilized powder is dissolved in sterile water at a concentration of 5 mg/ml, and the solution is administered to the patient after being nebulized by a nebulizer; two administrations every day with a dose of 10mg each time.

After 5 doses of administrations, the patient's blood pressure decreases from the previous 128/84 mmHg to 120/83 mmHg. It indicates that plasminogen can reduce blood pressure in a critical COVID-19 patient.

**Example 18: Plasminogen Reduces Blood Pressure and Respiratory Rate in Critical COVID-19 Patients**

**The patient:** a 47-year-old male with a blood oxygen saturation of 82% and a respiratory rate of 37 times/min, he is diagnosed with critical COVID-19 according to the clinical classification criteria of the Novel Coronavirus Pneumonia Diagnosis and Treatment Program (Trial Version 6). The patient's blood pressure is 139/94 mmHg (systolic/diastolic) before treatment.

Plasminogen lyophilized powder is dissolved in sterile water at a concentration of 5 mg/ml, and the solution is administered to the patient after being nebulized by a nebulizer; two administrations every day with a dose of 10mg each time.

After 2 doses of administrations, the patient's blood pressure decreases from the previous 139/94 mmHg to 120/83 mmHg, and the respiratory rate decreases from 37 times/min to 27 times/min. It indicates that plasminogen can reduce blood pressure and respiratory rate in a critical COVID-19 patient.

**Example 19: Plasminogen Improves the Condition of a COVID-19 Patient**

Thirteen 30-78 year-old COVID-19 patients are recruited in this experiment. According to the clinical classification criteria of the Novel Coronavirus Pneumonia Diagnosis and Treatment Program (Trial Version 6), these 13 patients include 5 ordinary patients, 6 severe patients, and 2 critical patients. The treatment is approved by the hospital ethics committee. All patients sign informed consent.

Human plasminogen lyophilized powder is dissolved in sterile water at a concentration of 5 mg/ml, and the solution is administered to the patients after being nebulized by a nebulizer. Different doses of plasminogen are administered according to the severity of the disease of the patients. Ordinary patients are administered once a day with a dose of 10 mg each time; severe and critical patients are administered twice a day with a dose of 10 mg each time. The basic circumstances of the patients and the frequency of administration are shown in Table 1. High-resolution chest CT (Neusoft, NeuViz 16Classic, China) is performed several days before administration or after the administration. The real-time blood oxygen saturation and heart rates are monitored by a monitor (Mindary, iPM5, China), and the blood oxygen saturation and heart rates are recorded 1 hour before aerosol inhalation of human plasminogen and several hours after the aerosol inhalation.

Table 1: Summary of human plasminogen therapy in COVID-19 patients

| ID number of patients | Sex | Age (years) | Clinical typing | Times of administration (times) |
|---|---|---|---|---|
| 1 | Male | 58 | Common type | 2 |
| 2 | Male | 30 | Common type | 2 |
| 3 | Female | 49 | Common type | 2 |
| 4 | Female | 48 | Common type | 3 |
| 5 | Male | 48 | Common type | 2 |
| 6 | Male | 46 | Severe type | 6 |
| 7 | Male | 78 | Severe type | 6 |
| 8 | Male | 47 | Severe type | 2 |
| 9 | Male | 65 | Severe type | 2 |
| 10 | Female | 56 | Severe type | 4 |
| 11 | Male | 78 | Severe type | 1 |
| 12 | Male | 48 | Critical type | 5 |
| 13 | Male | 47 | Critical type | 2 |

**Result 1: plasminogen improves lung injury in common COVID-19 patients**

CT results show that, the bilateral lungs in 5 patients of common type show multiple patchy/punctate "ground

glass" shadows with unclear boundary and uneven density before treatment, and the mediastinal window lesion regions show sparse spot-like shadows. Five patients received antibiotics and traditional Chinese medicine therapy before administration of human plasminogen, but the density and extent of the "ground glass" shadows in the lungs still increased over time, indicating aggravation of the disease. After 2-3 doses of human plasminogen administration, the number, extent and density of lung lesions in the 5 patients are reduced or even partially disappeared; patchy or punctate "ground glass" shadows are significantly reduced or absorbed (see Fig. 8 and Table 2). It indicates that aerosol inhalation of human plasminogen can rapidly alleviate lung injury caused by COVID-19 infection.

Table 2: High-resolution chest CT scan records of common COVID-19 patients

| ID number of patients | CT results before administrating plasminogen | CT results after administrating plasminogen |
|---|---|---|
| 1 | The bilateral lungs show multiple patchy/punctate "ground-glass" shadows with unclear boundary and uneven density, lung consolidation is observed in the right lower lobe. | The number and extent of lung lesions are decreased and partially disappeared. |
| 2 | The bilateral lungs show multiple patchy/punctate "ground-glass" shadows with unclear boundary and uneven density, lung consolidation is observed in the left lower lobe. | The lower lobes of two lungs show "ground glass" shadow, and the patchy dense region in the left lower lobe is smaller than that before treatment, and some of them are absorbed |
| 3 | The bilateral lungs show multiple patchy/punctate "ground-glass" shadows with unclear boundary and uneven density, mild lung consolidation is observed in the right lower lobe. | The number and area of lesions in the "cloud-like high-density" region of both lungs are smaller than those before treatment, and almost all of them are absorbed. |
| 4 | The bilateral lungs show multiple patchy/punctate "ground-glass" shadows with unclear boundary and uneven density, lung consolidation is observed in bilateral lower lobes. | The small patchy/ punctate "ground glass" shadows in the lower lobes of both lungs are smaller than those before treatment, and most of them are absorbed. |
| 5 | The bilateral lungs show multiple patchy/punctate "ground-glass" shadows with unclear boundary and uneven density, lung consolidation is observed in the middle or lower lobe of the right lung. | The patchy/punctate "ground glass" shadows in the right lower lobe are significantly reduced. |

**Result 2: plasminogen increases blood oxygen saturation in severe and critical COVID-19 patients**

During plasminogen administration, severe or critical COVID-19 patients are respectively infused with oxygen at a constant concentration of 80% or 100% through nasal cannula, and the blood oxygen saturation is monitored under oxygen infusion conditions.

The results show that, although blood oxygen saturation in severe patients is normal, after administration of human plasminogen, the blood oxygen saturation is still increased by 1-4% in 5 of the 6 severe patients. As for the 2 critical patients, only 1 hour after aerosol inhalation of human plasminogen, the blood oxygen saturation is increased from 79-82% before treatment to about 91%, and remained stable. After plasminogen is administered to one severe patient, the blood oxygen saturation is decreased from 91% before administration to 89% (Table 3). These data suggest that, plasminogen generally improves blood oxygen saturation levels in severe and critical COVID-19 patients, especially in patients with particularly low blood oxygen saturation.

Table 3: Blood oxygen saturation values of the severe and critical COVID-19 patients

| ID number of patients | Blood oxygen saturation before aerosol inhalation of plasminogen (%) | Blood oxygen saturation after aerosol inhalation of plasminogen (%) |
|---|---|---|
| 6 | 93 | 97 |
| 7 | 95 | 96 |
| 8 | 93 | 95 |

# EP 4 094 776 A1

(continued)

| ID number of patients | Blood oxygen saturation before aerosol inhalation of plasminogen (%) | Blood oxygen saturation after aerosol inhalation of plasminogen (%) |
|---|---|---|
| 9 | 93 | 96 |
| 10 | 91 | 95 |
| 11 | 91 | 89 |
| 12 | 79 | 91 |
| 13 | 82 | 91 |

**Result 3: Plasminogen can restore the heart rate of COVID-19 patients and reduce the burden on the heart**

The results of heart rate monitoring show that, after aerosol inhalation of human plasminogen, 8 of the 13 COVID-19 patients have their heart rate slowed down by approximately 26 beats per minute, 2 have an increased heart rate, and 3 have no significant change (Table 4). Statistical analysis shows that, after the administration of plasminogen, all the mean heart rates of the common, severe, and critical patients are decreased, and there is significant statistical difference in the comparison of the common patients before and after the administration of plasminogen ($p < 0.05$) (Fig. 9). These results suggest that, aerosol inhalation of human plasminogen generally slows the high heart rate in COVID-19 patients, and reduces the burden on the hearts.

Table 4: Results of heart rate monitoring in COVID-19 patients

| ID number of patients | Heat rate before administrating plasminogen (beats/min) | Heat rate after administrating (beats/min) |
|---|---|---|
| 1 | 92 | 76 |
| 2 | 84-104 | 80 |
| 3 | 82 | 82 |
| 4 | 82 | 82 |
| 5 | 84 | 76 |
| 6 | 98 | 72 |
| 7 | 81 | 70 |
| 8 | 73 | 75 |
| 9 | 73 | 73-75 |
| 10 | 79 | 78 |
| 11 | 71 | 72 |
| 12 | 92 | 70 |
| 13 | 110 | 89 |

In addition, all patients report reduced chest tightness and smoother breathing after aerosol inhalation of human plasminogen.

In conclusion, aerosol inhalation of human plasminogen can reduce lung damage in COVID-19 patients, increase blood oxygen saturation, reduce heart rate, and improve respiratory function in patients.

REFERENCES

[1] Yongzhi G, Jinan L, Elin H, et al. Beneficial and Detrimental Effects of Plasmin(ogen) during Infection and Sepsis in Mice[J]. PLoS ONE, 2011, 6(9):e24774-.

[2] Guo Y, Li J, Elin Hagström, et al. Protective effects of plasmin(ogen) in Staphylococcus aureus-induced arthritis[J]. Arthritis & Rheumatology, 2008, 58(3):764-772.

[3] Rubin Tan, Jiansha Li,Chuanyu Weil, Il-Kwon Kim, GAPDH is critical for superior efficacy of female bone marrow-derived mesenchymal stem cells on pulmonary hypertension, Cardiovascular Research (2013) 100, 19-27.

[4] Jose G. Gomez-Arroyo , The monocrotaline model of pulmonary hypertension in perspective , Am J Physiol Lung Cell Mol Physiol 302: L363-L369, 2012.

[5] Yosuke Kanno, En Shu, Hiroyuki Kanoh et al. The Antifibrotic Effect of a2AP Neutralization in Systemic Sclerosis Dermal Fibroblasts and Mouse Models of Systemic Sclerosis. J Invest Dermatol. 2016 Apr;136(4):762-9.

[6] Ming-wei Liu, Rong Liu,Hai-ying Wu et al.Radix puerariae extracts ameliorate paraquat-induced pulmonary fibrosis by attenuating follistatin-like 1 and nuclear factor erythroid 2p45-related factor-2signalling pathways through downregulation of miRNA-21 expression. BMC Complementary and Alternative Medicine (2016) 16:11.

[7] Franco R. D'Alessio. Mouse models of acute lung injury and ARDS[J]. 2018.

[8] Ambrus CM, Choi TS, Cunnanan E, Eisenberg B, Staub HP, Weintraub DH, et al. Prevention of hyaline membrane disease with plasminogen. A cooperative study. Jama. 1977; 237(17):1837-41.

SEQUENCE LISTING

SEQ ID NO: 1

gagcctctggatgactatgtgaatacccaggggggcttcactgttcagtgtcactaagaagcagctgggagcaggaagtatagaagaatgtgcagcaaaatgtgagga
ggacgaagaattcacctgcagggcattccaatatcacagtaaagagcaacaatgtgtgataatggctgaaaacaggaagtcctccataatcattaggatgagagatgt
agttttatttgaaaagaaagtgtatctctcagagtgcaagactgggaatggaaagaactacagagggacgatgtccaaaacaaaaaatggcatcacctgtcaaaaatg
gagttccacttctcccccacagacctagattctcacctgctacacaccccctcagagggactggaggagaactactgcaggaatccagacaacgatccgcaggggccct
ggtgctatactactgatccagaaaagagatatgactactgcgacattcttgagtgtgaagaggaatgtatgcattgcagtggagaaaactatgacggcaaaatttccaa
gaccatgtctggactggaatgccaggcctgggactctcagagcccacacgctcatggatacattccttccaaatttccaaacaagaacctgaagaagaattactgtcgt
aaccccgatagggagctgcggccttggtgtttcaccaccgaccccaacaagcgctgggaactttgtgacatcccccgctgcacaacacctccaccatcttctggtccc
acctaccagtgtctgaagggaacaggtgaaaactatcgcgggaatgtggctgttaccgtgtccgggcacacctgtcagcactggagtgcacagacccctcacacac
ataacaggacaccagaaaacttcccctgcaaaaatttggatgaaaactactgccgcaatcctgacggaaaaagggcccccatggtgccatacaaccaacagccaagt
gcggtggagtactgtaagataccgtcctgtgactcctcccccagtatccacggaacaattggctcccacagcaccacctgagctaacccctgtggtccaggactgcta
ccatggtgatggacagagctaccgaggcacatcctccaccaccaccacaggaaagaagtgtcagtcttggtcatctatgacaccacaccggcaccagaagaccccca
gaaaactacccaaatgctggcctgacaatgaactactgcaggaatccagatgccgataaaggcccctggtgtttaccacagaccccagcgtcaggtgggagtactg
caacctgaaaaaatgctcaggaacagaagcgagtgttgtagcacctccgcctgttgtcctgcttccagatgtagagactccttccgaagaagactgtatgtttgggaat
gggaaaggataccgaggcaagagggcgaccactgttactgggacgccatgccaggactgggctgcccaggagccccatagacacagcattttcactccagagac
aaatccacgggcgggtctggaaaaaaattactgccgtaaccctgatggtgatgtaggtggtccctggtgctacacgacaaatccaagaaaactttacgactactgtgat
gtccctcagtgtgcggcccccttcatttgattgtgggaagcctcaagtggagccgaagaaatgtcctggaagggttgtaggggggtgtgtggcccacccacattcctgg
ccctggcaagtcagtcttagaacaaggtttggaatgcacttctgtggaggcaccttgatatccccagagtgggtgttgactgctgcccactgcttggagaagtccccaa
ggccttcatcctacaaggtcatcctgggtgcacaccaagaagtgaatctcgaaccgcatgttcaggaaatagaagtgtctaggctgttcttggagcccacacgaaaag
atattgccttgctaaagctaagcagtcctgccgtcatcactgacaaagtaatcccagcttgtctgccatcccccaaattatgtggtcgctgaccggaccgaatgtttcatca
ctggctggggagaaacccaaggtacttttggagctggccttctcaaggaagcccagctccctgtgattgagaataaagtgtgcaatcgctatgagtttctgaatggaag
agtccaatccaccgaactctgtgctgggcatttggccggaggcactgacagttgccagggtgacagtggaggtcctctggtttgcttcgagaaggacaaatacatttta

caaggagtcacttcttggggtcttggctgtgcacgccccaataagcctggtgtctatgttcgtgtttcaaggtttgttacttggattgagggagtgatgagaaataattaa

SEQ ID NO: 2

EPLDDYVNTQGASLFSVTKKQLGAGSIEECAAKCEEDEEFTCRAFQYHSKEQQCVIMAENRKSSIIRMR
DVVLFEKKVYLSECKTGNGKNYRGTMSKTKNGITCQKWSSTSPHRPRFSPATHPSEGLEENYCRNPDND
PQGPWCYTTDPEKRYDYCDILECEEECMHCSGENYDGKISKTMSGLECQAWDSQSPHAHGYIPSKFPNK
NLKKNYCRNPDRELRPWCFTTDPNKRWELCDIPRCTTPPPSSGPTYQCLKGTGENYRGNVAVTVSGHTC
QHWSAQTPHTHNRTPENFPCKNLDENYCRNPDGKRAPWCHTTNSQVRWEYCKIPSCDSSPVSTEQLAPT
APPELTPVVQDCYHGDGQSYRGTSSTTTTGKKCQSWSSMTPHRHQKTPENYPNAGLTMNYCRNPDADK
GPWCFTTDPSVRWEYCNLKKCSGTEASVVAPPPVVLLPDVETPSEEDCMFGNGKGYRGKRATTVTGTP
CQDWAAQEPHRHSIFTPETNPRAGLEKNYCRNPDGDVGGPWCYTTNPRKLYDYCDVPQCAAPSFDCGK
PQVEPKKCPGRVVGGCVAHPHSWPWQVSLRTRFGMHFCGGTLISPEWVLTAAHCLEKSPRPSSYKVILG
AHQEVNLEPHVQEIEVSRLFLEPTRKDIALLKLSSPAVITDKVIPACLPSPNYVVADRTECFITGWGETQGT
FGAGLLKEAQLPVIENKVCNRYEFLNGRVQSTELCAGHLAGGTDSCQGDSGGPLVCFEKDKYILQGVTS
WGLGCARPNKPGVYVRVSRFVTWIEGVMRNN

SEQ ID NO: 3

atggaacataaggaagtggttcttctacttcttttatttctgaaatcaggtcaaggagagcctctggatgactatgtgaatacccaggggggcttcactgttcagtgtcactaa
gaagcagctgggagcaggaagtatagaagaatgtgcagcaaaatgtgaggaggacgaagaattcacctgcagggcattccaatatcacagtaaagagcaacaatg
tgtgataatggctgaaaacaggaagtcctccataatcattaggatgagagatgtagtttfatttgaaaagaaagtgtatctctcagagtgcaagactgggaatggaaaga
actacagagggacgatgtccaaaacaaaaaatggcatcacctgtcaaaaatggagttccacttctccccacagacctagattctcacctgctacacacccctcagagg
gactggaggagaactactgcaggaatccagacaacgatccgcagggccctggtgctatactactgatccagaaaagagatatgactactgcgacattcttgagtgt
gaagaggaatgtatgcattgcagtggagaaaactatgacggcaaaatttccaagaccatgtctggactggaatgccaggcctgggactctcagagcccacacgctca
tggatacattccttccaaatttccaaacaagaacctgaagaagaattactgtcgtaaccccgataggggagctgcggccttggtgtttcaccaccgaccccaacaagcgc
tgggaactttgtgacatcccccgctgcacaacacctccaccatcttctggtcccacctaccagtgtctgaagggaacaggtgaaaactatcgcgggaatgtggctgtta
ccgtgtccgggcacacctgtcagcactggagtgcacagacccctcacacacataacaggacaccagaaaacttcccctgcaaaaatttggatgaaaactactgccgc
aatcctgacggaaaaagggcccccatggtgccatacaaccaacagccaagtgcggtgggagtactgtaagataccgtcctgtgactcctccccagtatccacggaac
aattggctcccacagcaccacctgagctaacccctgtggtccaggactgctaccatggtgatggacagagctaccgaggcacatcctccaccaccaccacaggaaa
gaagtgtcagtcttggtcatctatgacaccacaccggcaccagaagaccccagaaaactacccaaatgctggcctgacaatgaactactgcaggaatccagatgccg
ataaaggcccctggtgttttaccacagaccccagcgtcaggtgggagtactgcaacctgaaaaaatgctcaggaacagaagcgagtgttgtagcacctccgcctgtt
gtcctgcttccagatgtagagactcctccgaagaagactgtatgtttgggaatgggaaaggataccgaggcaagagggcgaccactgttactgggacgccatgcca
ggactgggctgcccaggagcccccatagacacagcattttcactccagagacaaatccacgggcgggtctggaaaaaaattactgccgtaaccctgatggtgatgtag
gtggtccctggtgctacacgacaaatccaagaaaactttacgactactgtgatgtccctcagtgtgcggcccccttcatttgattgtgggaagcctcaagtggagccgaa
gaaatgtcctggaagggttgtaggggggtgtgtggcccacccacattcctggccctggcaagtcagtcttagaacaaggtttggaatgcacttctgtggaggcacctt
gatatccccagagtgggtgttgactgctgcccactgcttggagaagtccccaaggccttcatcctacaaggtcatcctgggtgcacaccaagaagtgaatctcgaacc
gcatgttcaggaaatagaagtgtctaggctgttcttggagcccacacgaaaagatattgccttgctaaagctaagcagtcctgccgtcatcactgacaaagtaatccca
gcttgtctgccatccccaaattatgtggtcgctgaccggaccgaatgtttcatcactggctggggagaaacccaaggtactttttggagctggccttctcaaggaagccc
agctccctgtgattgagaataaagtgtgcaatcgctatgagtttctgaatggaagagtccaatccaccgaactctgtgctggcatttggccggaggcactgacagttg
ccagggtgacagtggaggtcctctggtttgcttcgagaaggacaaatacattttacaaggagtcacttcttggggtcttggctgtgcacgcccccaataagcctggtgtct
atgttcgtgtttcaaggtttgttacttggattgagggagtgatgagaaataattaa

SEQ ID NO: 4

MEHKEVVLLLLLFLKSGQGEPLDDYVNTQGASLFSVTKKQLGAGSIEECAAKCEEDEEFTCRAFQYHSK

EQQCVIMAENRKSSIIRMRDVVLFEKKVYLSECKTGNGKNYRGTMSKTKNGITCQKWSSTSPHRPRFSP
ATHPSEGLEENYCRNPDNDPQGPWCYTTDPEKRYDYCDILECEEECMHCSGENYDGKISKTMSGLECQA
WDSQSPHAHGYIPSKFPNKLKKNYCRNPDRELRPWCFTTDPNKRWELCDIPRCTTPPPSSGPTYQCLKG
TGENYRGNVAVTVSGHTCQHWSAQTPHTHNRTPENFPCKNLDENYCRNPDGKRAPWCHTTNSQVRWE
YCKIPSCDSSPVSTEQLAPTAPPELTPVVQDCYHGDGQSYRGTSSTTTTGKKCQSWSSMTPHRHQKTPEN
YPNAGLTMNYCRNPDADKGPWCFTTDPSVRWEYCNLKKCSGTEASVVAPPPVVLLPDVETPSEEDCMF
GNGKGYRGKRATTVTGTPCQDWAAQEPHRHSIFTPETNPRAGLEKNYCRNPDGDVGGPWCYTTNPRKL
YDYCDVPQCAAPSFDCGKPQVEPKKCPGRVVGGCVAHPHSWPWQVSLRTRFGMHFCGGTLISPEWVLT
AAHCLEKSPRPSSYKVILGAHQEVNLEPHVQEIEVSRLFLEPTRKDIALLKLSSPAVITDKVIPACLPSPNY
VVADRTECFITGWGETQGTFGAGLLKEAQLPVIENKVCNRYEFLNGRVQSTELCAGHLAGGTDSCQGDS
GGPLVCFEKDKYILQGVTSWGLGCARPNKPGVYVRVSRFVTWIEGVMRNN

SEQ ID NO: 5

aaagtgtatctctcagagtgcaagactgggaatggaaagaactacagagggacgatgtccaaaacaaaaaatggcatcacctgtcaaaatggagttccacttctccc
cacagacctagattctcacctgctacacacccctcagagggactggaggagaactactgcaggaatccagacaacgatccgcagggccctggtgctatactactg
atccagaaaagagatatgactactgcgacattcttgagtgtgaagaggaatgtatgcattgcagtggagaaaactatgacggcaaaatttccaagaccatgtctggact
ggaatgccaggcctgggactctcagagcccacacgctcatggatacattccttccaaatttccaaacaagaacctgaagaagaattactgtcgtaaccccgataggga
gctgcggccttggtgtttcaccaccgaccccaacaagcgctgggaactttgtgacatccccgctgcacaacacctccaccatcttctggtcccacctaccagtgtctg
aagggaacaggtgaaaactatcgcgggaatgtggctgttaccgtgtccgggcacacctgtcagcactggagtgcacagacccctcacacacataacaggacacca
gaaaacttcccctgcaaaaatttggatgaaaactactgccgcaatcctgacggaaaaaagggccccatggtgccatacaaccaacagccaagtgcggtgggagtact
gtaagataccgtcctgtgactcctccccagtatccacggaacaattggctcccacagcaccacctgagctaaccctgtggtccaggactgctaccatggtgatggac
agagctaccgaggcacatcctccaccaccaccacaggaaagaagtgtcagtcttggtcatctatgacaccacaccggcaccagaagacccccagaaaactacccaa
atgctggcctgacaatgaactactgcaggaatccagatgccgataaaaggcccctggtgtttttaccacagacccccagcgtcaggtgggagtactgcaacctgaaaaaa
tgctcaggaacagaagcgagtgttgtagcacctccgcctgttgtcctgcttccagatgtagagactccttccgaagaagactgtatgtttgggaatgggaaaggatacc
gaggcaagagggcgaccactgttactgggacgccatgccaggactgggctgcccaggagccccatagacacagcattttcactccagagacaaatccacgggcg
ggtctggaaaaaaattactgccgtaaccctgatggtgatgtaggtggtccctggtgctacacgacaaatccaagaaaactttacgactactgtgatgtccctcagtgtgc
ggccccttcatttgattgtgggaagcctcaagtggagccgaagaaatgtcctggaaggggttgtaggggggtgtgtggcccacccacattcctggccctggcaagtca
gtcttagaacaaggtttggaatgcacttctgtggaggcaccttgatatccccagagtgggtgttgactgctgcccactgcttggagaagtccccaaggccttcatcctac
aaggtcatcctgggtgcacaccaagaagtgaatctcgaaccgcatgttcaggaaatagaagtgtctaggctgttcttggagcccacacgaaaagatattgccttgctaa
agctaagcagtcctgccgtcatcactgacaaagtaatcccagcttgtctgccatccccaaattatgtggtcgctgaccggaccgaatgtttcatcactggctggggaga
aacccaaggtactttttggagctggccttctcaaggaagcccagctccctgtgattgagaataaagtgtgcaatcgctatgagtttctgaatggaagagtccaatccaccg
aactctgtgctgggcatttggccggaggcactgacagttgccagggtgacagtggaggtcctctggtttgcttcgagaaggacaaatacattttacaaggagtcacttct
tggggtcttggctgtgcacgccccaataagcctggtgtctatgttcgtgtttcaaggtttgttacttggattgagggagtgatgagaaataattaa

SEQ ID NO: 6

KVYLSECKTGNGKNYRGTMSKTKNGITCQKWSSTSPHRPRFSPATHPSEGLEENYCRNPDNDPQGPWCY
TTDPEKRYDYCDILECEEECMHCSGENYDGKISKTMSGLECQAWDSQSPHAHGYIPSKFPNKLKKNYC
RNPDRELRPWCFTTDPNKRWELCDIPRCTTPPPSSGPTYQCLKGTGENYRGNVAVTVSGHTCQHWSAQT
PHTHNRTPENFPCKNLDENYCRNPDGKRAPWCHTTNSQVRWEYCKIPSCDSSPVSTEQLAPTAPPELTPV
VQDCYHGDGQSYRGTSSTTTTGKKCQSWSSMTPHRHQKTPENYPNAGLTMNYCRNPDADKGPWCFTT
DPSVRWEYCNLKKCSGTEASVVAPPPVVLLPDVETPSEEDCMFGNGKGYRGKRATTVTGTPCQDWAAQ
EPHRHSIFTPETNPRAGLEKNYCRNPDGDVGGPWCYTTNPRKLYDYCDVPQCAAPSFDCGKPQVEPKKC
PGRVVGGCVAHPHSWPWQVSLRTRFGMHFCGGTLISPEWVLTAAHCLEKSPRPSSYKVILGAHQEVNLE

PHVQEIEVSRLFLEPTRKDIALLKLSSPAVITDKVIPACLPSPNYVVADRTECFITGWGETQGTFGAGLLKE
AQLPVIENKVCNRYEFLNGRVQSTELCAGHLAGGTDSCQGDSGGPLVCFEKDKYILQGVTSWGLGCARP
NKPGVYVRVSRFVTWIEGVMRNN

**SEQ ID NO: 7**

gagcctctggatgactatgtgaatacccaggggggcttcactgttcagtgtcactaagaagcagctgggagcaggaagtatagaagaatgtgcagcaaaatgtgagga
ggacgaagaattcacctgcagggcattccaatatcacagtaaagagcaacaatgtgtgataatggctgaaaacaggaagtcctccataatcattaggatgagagatgt
agttttatttgaaaagaaagtgtatctctcagagtgcaagactgggaatggaaagaactacagagggacgatgtccaaaacaaaaaatggcatcacctgtcaaaatg
gagttccacttctccccacagacctagattctcacctgctacacacccctcagagggactggaggagaactactgcaggaatccagacaacgatccgcaggggccct
ggtgctatactactgatccagaaaagagatatgactactgcgacattcttgagtgtgaagaggcggcccccttcatttgattgtgggaagcctcaagtggagccgaagaa
atgtcctggaagggttgtagggggggtgtgtggcccacccacattcctggccctggcaagtcagtcttagaacaaggtttggaatgcacttctgtggaggcaccttgata
tccccagagtgggtgttgactgctgcccactgcttggagaagtccccaaggccttcatcctacaaggtcatcctgggtgcacaccaagaagtgaatctcgaaccgcat
gttcaggaaatagaagtgtctaggctgttcttggagcccacacgaaaagatattgccttgctaaagctaagcagtcctgccgtcatcactgacaaagtaatcccagcttg
tctgccatccccaaattatgtggtcgctgaccggaccgaatgtttcatcactggctggggagaaacccaaggtactttggagctggccttctcaaggaagcccagctc
cctgtgattgagaataaagtgtgcaatcgctatgagtttctgaatggaagagtccaatccaccgaactctgtgctgggcatttggccggaggcactgacagttgccagg
gtgacagtggaggtcctctggtttgcttcgagaaggacaaatacattttacaaggagtcacttcttggggtcttggctgtgcacgccccaataagcctggtgtctatgttc
gtgtttcaaggtttgttacttggattgagggagtgatgagaaataattaa

**SEQ ID NO: 8**

EPLDDYVNTQGASLFSVTKKQLGAGSIEECAAKCEEDEEFTCRAFQYHSKEQQCVIMAENRKSSIIRMR
DVVLFEKKVYLSECKTGNGKNYRGTMSKTKNGITCQKWSSTSPHRPRFSPATHPSEGLEENYCRNPDND
PQGPWCYTTDPEKRYDYCDILECEEAAPSFDCGKPQVEPKKCPGRVVGGCVAHPHSWPWQVSLRTRFG
MHFCGGTLISPEWVLTAAHCLEKSPRPSSYKVILGAHQEVNLEPHVQEIEVSRLFLEPTRKDIALLKLSSPA
VITDKVIPACLPSPNYVVADRTECFITGWGETQGTFGAGLLKEAQLPVIENKVCNRYEFLNGRVQSTELC
AGHLAGGTDSCQGDSGGPLVCFEKDKYILQGVTSWGLGCARPNKPGVYVRVSRFVTWIEGVMRNN

**SEQ ID NO: 9**

gtcaggtgggagtactgcaacctgaaaaaatgctcaggaacagaagcgagtgttgtagcacctccgcctgttgtcctgcttccagatgtagagactccttccgaagaa
gactgtatgtttgggaatgggaaaggataccgaggcaagagggcgaccactgttactgggacgccatgccaggactgggctgcccaggagccccatagacacag
catttcactccagagacaaatccacgggcgggtctggaaaaaaattactgccgtaaccctgatggtgatgtaggtggtccctggtgctacacgacaaatccaagaaa
actttacgactactgtgatgtccctcagtgtgcggcccccttcatttgattgtgggaagcctcaagtggagccgaagaaatgtcctggaagggttgtagggggggtgtgtg
gcccacccacattcctggccctggcaagtcagtcttagaacaaggtttggaatgcacttctgtggaggcaccttgatatccccagagtgggtgttgactgctgcccact
gcttggagaagtccccaaggccttcatcctacaaggtcatcctgggtgcacaccaagaagtgaatctcgaaccgcatgttcaggaaatagaagtgtctaggctgttctt
ggagcccacacgaaaagatattgccttgctaaagctaagcagtcctgccgtcatcactgacaaagtaatcccagcttgtctgccatccccaaattatgtggtcgctgac
cggaccgaatgtttcatcactggctggggagaaacccaaggtactttggagctggccttctcaaggaagcccagctccctgtgattgagaataaagtgtgcaatcgct
atgagtttctgaatggaagagtccaatccaccgaactctgtgctgggcatttggccggaggcactgacagttgccagggtgacagtggaggtcctctggtttgcttcga
gaaggacaaatacattttacaaggagtcacttcttggggtcttggctgtgcacgccccaataagcctggtgtctatgttcgtgtttcaaggtttgttacttggattgaggga
gtgatgagaaataattaa

**SEQ ID NO: 10**

VRWEYCNLKKCSGTEASVVAPPPVVLLPDVETPSEEDCMFGNGKGYRGKRATTVTGTPCQDWAAQEPH
RHSIFTPETNPRAGLEKNYCRNPDGDVGGPWCYTTNPRKLYDYCDVPQCAAPSFDCGKPQVEPKKCPGR
VVGGCVAHPHSWPWQVSLRTRFGMHFCGGTLISPEWVLTAAHCLEKSPRPSSYKVILGAHQEVNLEPHV

QEIEVSRLFLEPTRKDIALLKLSSPAVITDKVIPACLPSPNYVVADRTECFITGWGETQGTFGAGLLKEAQL
PVIENKVCNRYEFLNGRVQSTELCAGHLAGGTDSCQGDSGGPLVCFEKDKYILQGVTSWGLGCARPNKP
GVYVRVSRFVTWIEGVMRNN

**SEQ ID NO: 11**

gccccttcatttgattgtgggaagcctcaagtggagccgaagaaatgtcctggaagggttgtaggggggtgtgtggcccacccacattcctggccctggcaagtcagt
cttagaacaaggtttggaatgcacttctgtggaggcaccttgatatccccagagtgggtgttgactgctgcccactgcttggagaagtccccaaggccttcatcctacaa
ggtcatcctgggtgcacaccaagaagtgaatctcgaaccgcatgttcaggaaatagaagtgtctaggctgttcttggagcccacacgaaaagatattgccttgctaaag
ctaagcagtcctgccgtcatcactgacaaagtaatcccagcttgtctgccatccccaaattatgtggtcgctgaccggaccgaatgtttcatcactggctggggagaaa
cccaaggtacttttggagctggccttctcaaggaagcccagctccctgtgattgagaataaagtgtgcaatcgctatgagtttctgaatggaagagtccaatccaccgaa
ctctgtgctgggcatttggccggaggcactgacagttgccagggtgacagtggaggtcctctggtttgcttcgagaaggacaaatacattttacaaggagtcacttcttg
gggtcttggctgtgcacgccccaataagcctggtgtctatgttcgtgtttcaaggtttgttacttggattgagggagtgatgagaaataattaa

**SEQ ID NO: 12**

APSFDCGKPQVEPKKCPGRVVGGCVAHPHSWPWQVSLRTRFGMHFCGGTLISPEWVLTAAHCLEKSPRP
SSYKVILGAHQEVNLEPHVQEIEVSRLFLEPTRKDIALLKLSSPAVITDKVIPACLPSPNYVVADRTECFITG
WGETQGTFGAGLLKEAQLPVIENKVCNRYEFLNGRVQSTELCAGHLAGGTDSCQGDSGGPLVCFEKDK
YILQGVTSWGLGCARPNKPGVYVRVSRFVTWIEGVMRNN

**SEQ ID NO: 13**

gttgtaggggggtgtgtggcccacccacattcctggccctggcaagtcagtcttagaacaaggtttggaatgcacttctgtggaggcaccttgatatccccagagtggg
tgttgactgctgcccactgcttggagaagtccccaaggccttcatcctacaaggtcatcctgggtgcacaccaagaagtgaatctcgaaccgcatgttcaggaaataga
agtgtctaggctgttcttggagcccacacgaaaagatattgccttgctaaagctaagcagtcctgccgtcatcactgacaaagtaatcccagcttgtctgccatccccaa
attatgtggtcgctgaccggaccgaatgtttcatcactggctggggagaaacccaaggtacttttggagctggccttctcaaggaagcccagctccctgtgattgagaa
taaagtgtgcaatcgctatgagtttctgaatggaagagtccaatccaccgaactctgtgctgggcatttggccggaggcactgacagttgccagggtgacagtggagg
tcctctggtttgcttcgagaaggacaaatacattttacaaggagtcacttcttggggtcttggctgtgcacgccccaataagcctggtgtctatgttcgtgtttcaaggtttgt
tacttggattgagggagtgatgaga

**SEQ ID NO: 14**

VVGGCVAHPHSWPWQVSLRTRFGMHFCGGTLISPEWVLTAAHCLEKSPRPSSYKVILGAHQEVNLEPHV
QEIEVSRLFLEPTRKDIALLKLSSPAVITDKVIPACLPSPNYVVADRTECFITGWGETQGTFGAGLLKEAQL
PVIENKVCNRYEFLNGRVQSTELCAGHLAGGTDSCQGDSGGPLVCFEKDKYILQGVTSWGLGCARPNKP
GVYVRVSRFVTWIEGVMR

## Claims

1. A method for preventing and treating pneumonia, comprising: administrating to a subject a therapeutically effective amount of one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

2. The method according to claim 1, wherein the component of plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains and protease domains of plasminogen and plasmin, mini-plasminogen, mini-plasmin, micro-plasminogen, micro-plasmin, delta-plasminogen, delta-plasmin, an activator of plasminogen, tPA and uPA.

3. The method according to claim 1, wherein the antagonist of the fibrinolysis inhibitor is an inhibitor of PAI-1, complement C1 inhibitor, $\alpha$2 antiplasmin or $\alpha$2 macroglobulin, e.g., an antibody.

4. The method according to any one of claims 1-3, wherein the pneumonia is bacterial pneumonia, viral pneumonia, mycoplasmal pneumonia, chlamydia pneumonia, fungal pneumonia, or rickettsial pneumonia.

5. The method according to any one of claims 1-3, wherein the pneumonia is caused by a non-infectious factor.

6. The method according to claim 4, wherein the pneumonia is coronavirus pneumonia.

7. The method according to claim 6, wherein the pneumonia is 2019-nCoV infectious pneumonia, and the plasminogen has one or more effects selected from the group consisting of: reducing lung tissue damage, reducing lung inflammation, reducing lung fibrin deposition, improving lung function, increasing blood oxygen saturation, reducing blood pressure, improving cardiac function, and improving general physical condition in the subject with 2019-nCoV pneumonia.

8. The method according to any one of claims 1-7, wherein the compound has one or more effects selected from the group consisting of: reducing lung tissue inflammation, reducing lung tissue inflammatory exudation, reducing lung tissue fibrin deposition, reducing lung tissue fibrosis, reducing lung tissue apoptosis, improving ventilation function, and increasing blood oxygen saturation.

9. The method according to any one of claims 1-8, wherein the plasminogen has at least 75%, 80%, 85%, 90%, 95%, 96%, 97%, 98% or 99% sequence identity with SEQ ID NO: 2 and has plasminogen activity.

10. The method according to any one of claims 1-8, wherein the plasminogen comprises an amino acid sequence having at least 75%, 80%, 85%, 90%, 95%, 96%, 97%, 98% or 99% sequence identity with the active fragment of plasminogen represented by SEQ ID NO: 14, and has the proteolytic activity or lysine binding activity of plasminogen.

11. The method according to any one of claims 1-8, wherein the plasminogen is a conservative substitution variant of the plasminogen of SEQ ID NO: 2.

12. The method according to any one of claims 1-8, wherein the plasminogen is natural or synthetic human plasminogen.

13. The method according to any one of claims 1-12, wherein the compound is used in combination with one or more other therapeutic methods or medicaments.

14. The method according to claim 13, wherein the other medicament is one or more medicaments selected from the group consisting of: antiviral medicament, antibiotic, immunomodulator, hormonal medicament (e.g., steroid hormone), vaccine, and disease-associated neutralizing antibody.

15. The method according to any one of claims 1-14, wherein the compound is administered by one or more routes or means selected from the group consisting of: nasal inhalation, aerosol inhalation, nasal drop, ear drop, eye drop, intravenous administration, intraperitoneal administration, subcutaneous administration, intracranial administration, intrathecal administration, intramuscular administration and intrarectal administration.

Figure 1

Figure 2

Figure 3

Figure 4

EP 4 094 776 A1

Figure 5

Figure 6

Figure 7

30

Figure 8

● Before administrating plasminogen
■ After administrating plasminogen

Common type   Severe type   Critical type

Figure 9

| INTERNATIONAL SEARCH REPORT | International application No. |
|---|---|
| | **PCT/CN2021/076035** |

| **A.** | **CLASSIFICATION OF SUBJECT MATTER** |
|---|---|

A61K 38/48(2006.01)i; A61K 38/49(2006.01)i; A61K 38/36(2006.01)i; A61P 31/00(2006.01)i; A61P 31/14(2006.01)i; A61P 11/00(2006.01)i

According to International Patent Classification (IPC) or to both national classification and IPC

| **B.** | **FIELDS SEARCHED** |
|---|---|

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, USTXT, WOTXT, CNKI, 万方, WANFANG, EBI, NCBI, 百度, BAIDU, ISI Web of Knowledge, PUBMED: 泰伦基国际有限公司, 李季男, 肺炎, Pneumonia, Plasminogen, tPA, uPA, PAI-1, Coronavirus, 纤维蛋白溶酶原, 纤维蛋白溶酶, 纤溶酶, 纤溶酶原, 纤溶抑制剂的拮抗剂, PAI-1, 补体C1抑制物, α2抗纤溶酶, α2巨球蛋白, 抑制剂, 2019-nCoV, 冠状病毒, COVID-19

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | WANG, J. et al. "Tissue Plasminogen Activator (tPA) Treatment for COVID-19 Associated Acute Respiratory Distress Syndrome (ARDS): A Case Series" *J Thromb Haemost.*, Vol. 7, No. 18, 11 May 2020 (2020-05-11), pp. 1-4 | 1-15 |
| PX | CHOUDHURY, R. et al. "Salvage Use of Tissue Plasminogen Activator (tPA) in the Setting of Acute Respiratory Distress Syndrome (ARDS) due to COVID-19 in the USA: A Markov Decision Analysis" *World Journal of Emergency Surgery*, Vol. 29, No. 15, 20 April 2020 (2020-04-20), pp. 1-6 | 1-15 |
| PX | WU, Y. et al. "Plasminogen Improves Lung Lesions and Hypoxemia in Patients with COVID-19" *QJM*, Vol. 8, No. 113, 10 April 2020 (2020-04-10), pp. 539-544 | 1-15 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 April 2021** | **25 April 2021** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/076035**

**C.    DOCUMENTS CONSIDERED TO BE RELEVANT**

| Category* | Citation of document, with indication, where appropriate, of the relevant passages | Relevant to claim No. |
|---|---|---|
| PX | MOORE, H. B. et al. "Is There a Role for Tissue Plasminogen Activator as a Novel Treatment for Refractory COVID-19 Associated Acute Respiratory Distress Syndrome" *Journal of Trauma and Acute Care Surgery*, Vol. 6, No. 88, 20 March 2020 (2020-03-20), pp. 713-714 | 1-15 |
| X | CN 101573134 A (OMNIO HEALER AB) 04 November 2009 (2009-11-04) claims 1-22, and description, pages 10-19, 22 and 23 | 1-15 |
| A | WO 2018107694 A1 (TALENGEN INSTITUTE OF LIFE SCIENCES, CO. LTD.) 21 June 2018 (2018-06-21) entire document | 1-15 |
| A | US 2008214682 A1 (WASHINGTON UNIVERSITY IN ST. LOUIS) 04 September 2008 (2008-09-04) entire document | 1-15 |
| A | CN 107405379 A (THE BOARD OF REGENTS, THE UNIVERSITY OF TEXAS SYSTEM) 28 November 2017 (2017-11-28) entire document | 1-15 |
| A | CN 105705520 A (SANOFI) 22 June 2016 (2016-06-22) entire document | 1-15 |

Form PCT/ISA/210 (second sheet) (January 2015)

**EP 4 094 776 A1**

<table>
<tr><td colspan="2" align="center">**INTERNATIONAL SEARCH REPORT**</td><td>International application No.<br>**PCT/CN2021/076035**</td></tr>
</table>

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2021/076035** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **1-15**
   because they relate to subject matter not required to be searched by this Authority, namely:

   [1]  Claims 1-15 relate to methods for the prevention and treatment of pneumonia and therefore do not comply with PCT Rule 39.1(iv). This report is made on the basis of claims 1-15 modified to claims for pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

<table>
<tr><td colspan="2" align="center">**INTERNATIONAL SEARCH REPORT**<br>Information on patent family members</td><td colspan="2">International application No.<br><br>**PCT/CN2021/076035**</td></tr>
</table>

| Patent document cited in search report | | | Publication date (day/month/year) | Patent family member(s) | | | Publication date (day/month/year) |
|---|---|---|---|---|---|---|---|
| CN | 101573134 | A | 04 November 2009 | CN | 101563100 | B | 07 August 2013 |
| | | | | CA | 2662101 | A1 | 06 March 2008 |
| | | | | EP | 2056864 | A2 | 13 May 2009 |
| | | | | KR | 20090059122 | A | 10 June 2009 |
| | | | | US | 2013149321 | A1 | 13 June 2013 |
| | | | | CN | 101573134 | B | 06 March 2013 |
| | | | | EP | 2056864 | A4 | 25 January 2012 |
| | | | | JP | 2010502600 | A | 28 January 2010 |
| | | | | JP | 5566105 | B2 | 06 August 2014 |
| | | | | AU | 2007290881 | B2 | 07 March 2013 |
| | | | | US | 10729750 | B2 | 04 August 2020 |
| | | | | KR | 101517626 | B1 | 07 May 2015 |
| | | | | DK | 2056864 | T3 | 10 March 2014 |
| | | | | EA | 200970233 | A1 | 28 August 2009 |
| | | | | EP | 2056864 | B1 | 11 December 2013 |
| | | | | CA | 2662101 | C | 07 July 2015 |
| | | | | EA | 016250 | B1 | 30 March 2012 |
| | | | | WO | 2008026999 | A2 | 06 March 2008 |
| | | | | ES | 2451015 | T3 | 26 March 2014 |
| | | | | AU | 2007290881 | A1 | 06 March 2008 |
| | | | | US | 8318661 | B2 | 27 November 2012 |
| | | | | WO | 2008026999 | A3 | 22 May 2008 |
| | | | | US | 2016243204 | A1 | 25 August 2016 |
| | | | | MX | 2009002226 | A | 07 September 2009 |
| | | | | CN | 101563100 | A | 21 October 2009 |
| | | | | US | 2010099600 | A1 | 22 April 2010 |
| WO | 2018107694 | A1 | 21 June 2018 | CA | 3046669 | A1 | 21 June 2018 |
| | | | | CN | 110191718 | A | 30 August 2019 |
| | | | | TW | 201822796 | A | 01 July 2018 |
| | | | | CN | 110121358 | A | 13 August 2019 |
| | | | | EP | 3556382 | A4 | 09 December 2020 |
| | | | | CN | 110139668 | A | 16 August 2019 |
| | | | | TW | 201822783 | A | 01 July 2018 |
| | | | | CA | 3047174 | A1 | 21 June 2018 |
| | | | | EP | 3556380 | A4 | 13 May 2020 |
| | | | | EP | 3556395 | A4 | 22 July 2020 |
| | | | | WO | 2018107693 | A1 | 21 June 2018 |
| | | | | TW | I657823 | B | 01 May 2019 |
| | | | | EP | 3556382 | A1 | 23 October 2019 |
| | | | | JP | 2020512288 | A | 23 April 2020 |
| | | | | JP | 2020502132 | A | 23 January 2020 |
| | | | | TW | 201822795 | A | 01 July 2018 |
| | | | | EP | 3556395 | A1 | 23 October 2019 |
| | | | | EP | 3556380 | A1 | 23 October 2019 |
| | | | | US | 2019365872 | A1 | 05 December 2019 |
| | | | | EP | 3556389 | A4 | 15 July 2020 |
| | | | | TW | 201822790 | A | 01 July 2018 |
| | | | | CN | 110114080 | A | 09 August 2019 |
| | | | | WO | 2018107696 | A1 | 21 June 2018 |
| | | | | US | 2019314468 | A1 | 17 October 2019 |

Form PCT/ISA/210 (patent family annex) (January 2015)

International application No.

**PCT/CN2021/076035**

| Patent document cited in search report | | | Publication date (day/month/year) | Patent family member(s) | | | Publication date (day/month/year) |
|---|---|---|---|---|---|---|---|
| | | | | US | 2019314467 | A1 | 17 October 2019 |
| | | | | TW | 201822789 | A | 01 July 2018 |
| | | | | CA | 3046666 | A1 | 21 June 2018 |
| | | | | WO | 2018107697 | A1 | 21 June 2018 |
| | | | | US | 2019314466 | A1 | 17 October 2019 |
| | | | | WO | 2018107698 | A1 | 21 June 2018 |
| | | | | EP | 3556389 | A1 | 23 October 2019 |
| | | | | JP | 2020511413 | A | 16 April 2020 |
| | | | | CA | 3046671 | A1 | 21 June 2018 |
| | | | | TW | I670075 | B | 01 September 2019 |
| | | | | JP | 2020502133 | A | 23 January 2020 |
| US | 2008214682 | A1 | 04 September 2008 | | None | | |
| CN | 107405379 | A | 28 November 2017 | JP | 2018507867 | A | 22 March 2018 |
| | | | | CA | 2977519 | A1 | 01 September 2016 |
| | | | | AU | 2016225111 | A1 | 31 August 2017 |
| | | | | HK | 1247111 | A1 | 21 September 2018 |
| | | | | KR | 20170122748 | A | 06 November 2017 |
| | | | | BR | 112017018209 | A2 | 17 April 2018 |
| | | | | US | 2020164028 | A1 | 28 May 2020 |
| | | | | US | 2018050084 | A1 | 22 February 2018 |
| | | | | WO | 2016138413 | A1 | 01 September 2016 |
| | | | | EP | 3261657 | A1 | 03 January 2018 |
| CN | 105705520 | A | 22 June 2016 | CN | 105705520 | B | 25 September 2020 |
| | | | | MX | 2016001851 | A | 16 May 2016 |
| | | | | CL | 2016000324 | A1 | 21 October 2016 |
| | | | | US | 2016200831 | A1 | 14 July 2016 |
| | | | | US | 9845363 | B2 | 19 December 2017 |
| | | | | ES | 2770507 | T3 | 01 July 2020 |
| | | | | JP | 6696899 | B2 | 20 May 2020 |
| | | | | TN | 2016000048 | A1 | 05 July 2017 |
| | | | | EA | 201690377 | A1 | 30 June 2016 |
| | | | | CN | 112142845 | A | 29 December 2020 |
| | | | | SG | 10201710013R | A | 30 January 2018 |
| | | | | UA | 118267 | C2 | 26 December 2018 |
| | | | | JP | 2016529255 | A | 23 September 2016 |
| | | | | PH | 12016500239 | A1 | 16 May 2016 |
| | | | | JP | 2020125333 | A | 20 August 2020 |
| | | | | CR | 20160117 | A | 20 May 2016 |
| | | | | HK | 1221725 | A1 | 09 June 2017 |
| | | | | US | 2019359729 | A1 | 28 November 2019 |
| | | | | CR | 20190319 | A | 29 August 2019 |
| | | | | MX | 2019008803 | A | 16 September 2019 |
| | | | | EA | 033403 | B1 | 31 October 2019 |
| | | | | US | 2018155444 | A1 | 07 June 2018 |
| | | | | PE | 20160244 | A1 | 10 May 2016 |
| | | | | BR | 112016002753 | A2 | 21 November 2017 |
| | | | | KR | 20160035077 | A | 30 March 2016 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102154253 A
- WO 9704801 A

- US 3773919 A
- EP 58481 A

**Non-patent literature cited in the description**

- **CUI J ; LI F ; SHI Z L.** Origin and evolution of pathogenic coronaviruses[J]. Naturereviews. *Micorbiology,* 2019, vol. 17 (3), 181-192
- **WUHAN, HUBEI ; WHO.** Province and spread rapidly in a short period of time. *It was officially named 2019-nCoV,* December 2019
- **CHEN Y ; LIU Q ; GUO D.** Emerging coronaviruses: genome structure, replication, and pathogenesis [J. *Journal of medical virology,* 2020
- **SHI HESHUI ; HAN XIAOYU ; FAN YANQING ; LIANG BO ; YANG FAN ; HAN PING ; ZHENG CHUANSHENG.** Clinical features and imaging manifestations of pneumonia caused by novel coronavirus. *Journal of Clinical Radiology,* 2019, https://doi.org/10.13437/j.cnki.jcr.20200206.002
- **NY, A. ; LEONARDSSON, G. ; HAGGLUND, A.C ; HAGGLOF, P. ; PLOPLIS, V.A. ; CARMELIET, P. ; NY, T.** Ovulation in plasminogen-deficient mice. *Endocrinology,* 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.,* November 1984, vol. 74 (5), 1625-33
- **GRAVANIS I ; TSIRKA SE.** Tissue-type plasminogen activator as a therapeutic target in stroke. *Expert Opinion on Therapeutic Targets,* February 2008, vol. 12 (2), 159-70
- **GEIGER M ; HUBER K ; WOJTA J ; STINGL L ; ESPANA F ; GRIFFIN JH ; BINDER BR.** Complex formation between urokinase and plasma protein C inhibitor in vitro and in vivo. *Blood,* August 1989, vol. 74 (2), 722-8
- **AISINA RB ; MUKHAMETOVA L I.** Structure and function of plasminogen/plasmin system [J. *Russian Journal of Bioorganic Chemistry,* 2014, vol. 40 (6), 590-605
- Special Methods in Peptide Synthesis. *The Peptides: Analysis, Synthesis, Biology,* vol. 2, 3-284
- **MERRIFIELD et al.** *J. Am. Chem. Soc.,* 1963, vol. 85, 2149-2156

- **STEWART et al.** Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGE et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **KENNETH C ROBBINS ; LOUIS SUMMARIA ; DAVID ELWYN et al.** Further Studies on the Purification and Characterization of Human Plasminogen and Plasmin. *Journal of Biological Chemistry,* 1965, vol. 240 (1), 541-550
- **SUMMARIA L ; SPITZ F ; ARZADON L et al.** Isolation and characterization of the affinity chromatography forms of human Glu-and Lys-plasminogens and plasmins. *J Biol Chem.,* 25 June 1976, vol. 251 (12), 3693-9
- **HAGAN JJ ; ABLONDI FB ; DE RENZO EC.** Purification and biochemical properties of human plasminogen. *J Biol Chem.,* April 1960, vol. 235, 1005-10
- **YONGZHI G ; JINAN L ; ELIN H et al.** Beneficial and Detrimental Effects of Plasmin(ogen) during Infection and Sepsis in Mice[J. *PLoS ONE,* 2011, vol. 6 (9), e24774
- **GUO Y ; LI J ; ELIN HAGSTRÖM et al.** Protective effects of plasmin(ogen) in Staphylococcus aureus-induced arthritis[J. *Arthritis & Rheumatology,* 2008, vol. 58 (3), 764-772
- **RUBIN TAN ; JIANSHA LI ; CHUANYU WEIL ; IL-KWON KIM.** GAPDH is critical for superior efficacy of female bone marrow-derived mesenchymal stem cells on pulmonary hypertension. *Cardiovascular Research,* 2013, vol. 100, 19-27

- **JOSE G ; GOMEZ-ARROYO.** The monocrotaline model of pulmonary hypertension in perspective. *Am J Physiol Lung Cell Mol Physiol,* 2012, vol. 302, L363-L369
- **YOSUKE KANNO ; EN SHU ; HIROYUKI KANOH et al.** The Antifibrotic Effect of a2AP Neutralization in Systemic Sclerosis Dermal Fibroblasts and Mouse Models of Systemic Sclerosis. *J Invest Dermatol.,* April 2016, vol. 136 (4), 762-9
- **MING-WEI LIU ; RONG LIU ; HAI-YING WU et al.** Radix puerariae extracts ameliorate paraquat-induced pulmonary fibrosis by attenuating follistatin-like 1 and nuclear factor erythroid 2p45-related factor-2signalling pathways through downregulation of miRNA-21 expression. *BMC Complementary and Alternative Medicine,* 2016, vol. 16, 11
- **FRANCO R ; D'ALESSIO.** *Mouse models of acute lung injury and ARDS[J,* 2018
- **AMBRUS CM ; CHOI TS ; CUNNANAN E ; EISENBERG B ; STAUB HP ; WEINTRAUB DH et al.** Prevention of hyaline membrane disease with plasminogen. A cooperative study. *Jama,* 1977, vol. 237 (17), 1837-41